(19) Europäisches Patentamt
European Patent Office
Office européen des brevets

(11) EP 1 549 337 B1

(12) **EUROPEAN PATENT SPECIFICATION**

(45) Date of publication and mention of the grant of the patent:
**13.08.2014 Bulletin 2014/33**

(21) Application number: **03739577.9**

(22) Date of filing: **14.02.2003**

(51) Int Cl.:
*A61P 43/00* (2006.01) *A61K 38/46* (2006.01)
*A61K 31/726* (2006.01) *A61K 31/727* (2006.01)
*A61K 31/728* (2006.01) *A61K 31/737* (2006.01)

(86) International application number:
**PCT/GB2003/000668**

(87) International publication number:
**WO 2003/068254 (21.08.2003 Gazette 2003/34)**

(54) **Combination of DNase I and glycosaminoglycans for use in extracellular DNA clearance**

Kombination von DNase I und Glykosaminoglykanen für extrazellulären DNA-Abbau

Combinaison de DNase I et de glycosaminoglycanes pour leur utilisation dans l'élimination de l'ADN extracellulaire

(84) Designated Contracting States:
**AT BE BG CH CY CZ DE DK EE ES FI FR GB GR HU IE IT LI LU MC NL PT SE SI SK TR**

(30) Priority: **18.02.2002 GB 0203830**
**18.02.2002 GB 0203773**
**17.05.2002 GB 0211417**
**18.10.2002 GB 0224329**

(43) Date of publication of application:
**06.07.2005 Bulletin 2005/27**

(73) Proprietor: **Ockham Biotech Limited**
**Swanwick**
**Hampshire SO31 7HA (GB)**

(72) Inventors:
- **SHUTE, Janis Kay**
  **Portsmouth,**
  **Hampshire P06 2QP (GB)**
- **CARROLL, Mary Patricia,**
  **Adult Cystic Fibrosis Unit**
  **Southampton SO16 6YD (GB)**
- **Dr. CONWAY, Joy**
  **Hampshire SO51 8DB (GB)**
- **HOCKEY, Peter Morey**
  **Lymington,**
  **Hampshire SO41 5TB (GB)**

(74) Representative: **Woods, Geoffrey Corlett**
**J A Kemp**
**14 South Square**
**Gray's Inn**
**London WC1R 5JJ (GB)**

(56) References cited:
**EP-A- 1 074 260** **WO-A-01/15672**
**WO-A-95/23613** **WO-A-98/10053**
**GB-A- 1 560 463** **US-A- 5 633 003**
**US-B1- 6 235 725**

- **SHAK S ET AL: "Recombinant human DNase I reduces the viscosity of cystic fibrosis sputum", PROCEEDINGS OF THE NATIONAL ACADEMY OF SCIENCES, NATIONAL ACADEMY OF SCIENCES, US, vol. 87, no. 23, 1 December 1990 (1990-12-01), pages 9188-9192, XP002216655, ISSN: 0027-8424, DOI: 10.1073/PNAS.87.23.9188**
- **RANASINHA C ET AL: "EFFICACY AND SAFETY OF SHORT-TERM ADMINISTRATION OF AEROSOLISED RECOMBINANT HUMAN DNASE I IN ADULTS WITH STABLE STAGE CYSTIC FIBROSIS", THE LANCET, LANCET LIMITED. LONDON, GB, vol. 342, no. 8865, 24 July 1993 (1993-07-24), pages 199-202, XP002008763, ISSN: 0140-6736, DOI: 10.1016/0140-6736(93)92297-7**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 549 337 B1

SEQUENCE LISTING

<110>

<120> GLYCOSAMINOGLYCAN COMBINATION THERAPY

<130> P.85643 GCW

<140>
<141>

<160> 2

<170> PatentIn version 3.1

<210> 1
<211> 1055
<212> DNA
<213> Homo Sapiens

<220>
<221> CDS
<222> (2)..(1054)
<223>

<400> 1

```
a gcc atg cac tac cca act gca ctc ctc ttc ctc atc ctg gcc aat ggg      49
  Ala Met His Tyr Pro Thr Ala Leu Leu Phe Leu Ile Leu Ala Asn Gly
  1               5                   10                  15
acc cag acc ttt cgc atc tgc gcc ttc aat gcc cag cgg ctg aca ctg        97
Thr Gln Thr Phe Arg Ile Cys Ala Phe Asn Ala Gln Arg Leu Thr Leu
            20                  25                  30
ccc aag gtg gcc agg gag cag gtg atg gac acc tta gtt cgg ata ctg       145
Pro Lys Val Ala Arg Glu Gln Val Met Asp Thr Leu Val Arg Ile Leu
        35                  40                  45
gct cgc tgt gac atc atg gtg ctg cag gag gtg gtg gac tct tcc ggc       193
Ala Arg Cys Asp Ile Met Val Leu Gln Glu Val Val Asp Ser Ser Gly
    50                  55                  60
agc gcc atc ccg ctc ctg ctt cga gaa ctc aat cga ttt gat ggc tct       241
Ser Ala Ile Pro Leu Leu Leu Arg Glu Leu Asn Arg Phe Asp Gly Ser
65                  70                  75                  80
ggg ccc tac agc acc ctg agc agc ccc cag ctg ggg cgc agc acc tac       289
Gly Pro Tyr Ser Thr Leu Ser Ser Pro Gln Leu Gly Arg Ser Thr Tyr
                85                  90                  95
atg gag acg tat gtg tac ttc tat cgg tca cac aaa aca cag gtc ctg       337
Met Glu Thr Tyr Val Tyr Phe Tyr Arg Ser His Lys Thr Gln Val Leu
            100                 105                 110
agt tcc tac gtg tac aac gat gag gat gac gtc ttt gcc cgg gag cca       385
Ser Ser Tyr Val Tyr Asn Asp Glu Asp Asp Val Phe Ala Arg Glu Pro
        115                 120                 125
ttt gtg gcc cag ttc tct ttg ccc agc aat gtc ctt ccc agc ctg gtg       433
Phe Val Ala Gln Phe Ser Leu Pro Ser Asn Val Leu Pro Ser Leu Val
    130                 135                 140
ttg gtc ccg ctg cac acc act cct aag gcc gta gag aag gag ctg aac       481
Leu Val Pro Leu His Thr Thr Pro Lys Ala Val Glu Lys Glu Leu Asn
145                 150                 155                 160
```

```
gcc ctc tac gat gtg ttt ctg gag gtc tcc cag cac tgg cag agc aag      529
Ala Leu Tyr Asp Val Phe Leu Glu Val Ser Gln His Trp Gln Ser Lys
                165                 170                 175
gac gtg atc ctg ctt ggg gac ttc aat gct gac tgc gct tca ctg acc      577
Asp Val Ile Leu Leu Gly Asp Phe Asn Ala Asp Cys Ala Ser Leu Thr
            180                 185                 190
aaa aag cgc ctg gac aag ctg gag ctg cgg act gag cca ggc ttc cac      625
Lys Lys Arg Leu Asp Lys Leu Glu Leu Arg Thr Glu Pro Gly Phe His
        195                 200                 205
tgg gtg att gcc gat ggg gag gac acc aca gtg cgg gcc agc acc cac      673
Trp Val Ile Ala Asp Gly Glu Asp Thr Thr Val Arg Ala Ser Thr His
    210                 215                 220
tgc acc tat gac cgc gtc gtg ctg cac ggg gag cgc tgc cgg agt ctg      721
Cys Thr Tyr Asp Arg Val Val Leu His Gly Glu Arg Cys Arg Ser Leu
225                 230                 235                 240
ctg cac act gcg gct gcc ttt gac ttc ccc acg agc ttc cag ctc acc      769
Leu His Thr Ala Ala Ala Phe Asp Phe Pro Thr Ser Phe Gln Leu Thr
                245                 250                 255
gag gag gag gcc ctc aac atc agt gac cac tac ccc gtg gag gtg gag      817
Glu Glu Glu Ala Leu Asn Ile Ser Asp His Tyr Pro Val Glu Val Glu
            260                 265                 270
ctg aag ctg agc cag gcg cac agc gtc cag cct ctc agc ctc act gtt      865
Leu Lys Leu Ser Gln Ala His Ser Val Gln Pro Leu Ser Leu Thr Val
        275                 280                 285
ctg ttg ctg cta tca ctc ctg tcc cct cag ctg tgc cct gct gcc tga      913
Leu Leu Leu Leu Ser Leu Leu Ser Pro Gln Leu Cys Pro Ala Ala
    290                 295                 300
gcg tcc ccc tac ccc ccc agg gcc tgc tgc ctt ttg gga ctt aaa ccc      961
cag cct ccc ccg tcc atc cag ccc tgg ggc tgg ggg gct tca act ata     1009
gtt gcc ctg tga ctg tag tcc acc cct gcc tgc ctt gtt tga ttt g       1055
```

<210> 2
<211> 351
<212> PRT
<213> Homo sapiens

<400> 2

Ala Met His Tyr Pro Thr Ala Leu Leu Phe Leu Ile Leu Ala Asn Gly
1 5 10 15
Thr Gln Thr Phe Arg Ile Cys Ala Phe Asn Ala Gln Arg Leu Thr Leu
20 25 30
Pro Lys Val Ala Arg Glu Gln Val Met Asp Thr Leu Val Arg Ile Leu
35 40 45
Ala Arg Cys Asp Ile Met Val Leu Gln Glu Val Val Asp Ser Ser Gly
50 55 60
Ser Ala Ile Pro Leu Leu Leu Arg Glu Leu Asn Arg Phe Asp Gly Ser
65 70 75 80
Gly Pro Tyr Ser Thr Leu Ser Ser Pro Gln Leu Gly Arg Ser Thr Tyr
85 90 95
Met Glu Thr Tyr Val Tyr Phe Tyr Arg Ser His Lys Thr Gln Val Leu
100 105 110
Ser Ser Tyr Val Tyr Asn Asp Glu Asp Asp Val Phe Ala Arg Glu Pro
115 120 125
Phe Val Ala Gln Phe Ser Leu Pro Ser Asn Val Leu Pro Ser Leu Val
130 135 140
Leu Val Pro Leu His Thr Thr Pro Lys Ala Val Glu Lys Glu Leu Asn

145 150 155 160
Ala Leu Tyr Asp Val Phe Leu Glu Val Ser Gln His Trp Gln Ser Lys
165 170 175
Asp Val Ile Leu Leu Gly Asp Phe Asn Ala Asp Cys Ala Ser Leu Thr
180 185 190
Lys Lys Arg Leu Asp Lys Leu Glu Leu Arg Thr Glu Pro Gly Phe His
195 200 205
Trp Tyr Ile Ala Asp Gly Glu Asp Thr Thr Val Arg Ala Ser Thr His
210 215 220
Cys Thr Tyr Asp Arg Val Val Leu His Gly Glu Arg Cys Arg Ser Leu
225 230 235 240
Leu His Thr Ala Ala Ala Phe Asp Phe Pro Thr Ser Phe Gln Leu Thr
245 250 255
Glu Glu Glu Ala Leu Asn Ile Ser Asp His Tyr Pro Val Glu Val Glu
260 265 270
Leu Lys Leu Ser Gln Ala His Ser Val Gln Pro Leu Ser Leu Thr Val
275 280 285
Leu Leu Leu Leu Ser Leu Leu Ser Pro Gln Leu Cys Pro Ala Ala
290 295 300

## Description

### Field of Invention

The present invention is described in claims 1-21 and relates to DNase I and in particular to the use of DNase I in the treatment of disorders characterised by the presence of endogenous extracellular DNA.

### Background of Invention

DNases are a group of phosphodiesterase enzymes capable of hydrolysing DNA. They act in a non-specific manner, as they do not require the presence of a specific sequence in the target molecule and are capable of extensively degrading DNA molecules cleaving them multiple times. The two main types of DNases are DNase I and DNase II type enzymes. There are also DNase type III enzymes. DNase I requires cations to be active and has a pH optimum of near neutral. It hydrolyses DNA to produce 5'-phosphate nucleotides. DNase II can be activated by divalent cations and has an acidic pH optimum. It hydrolyses DNA to produce 3'-phosphate nucleotides.
Clinically DNases, and in particular DNase I, are mainly thought useful in the treatment of respiratory disorders and are presently used to treat cystic fibrosis. It has been suggested that DNases may also be useful in the treatment of a number of other conditions including those involving enclosed inflammatory sites and autoimmune disorders such as Systemic Lupus Erythematosus (SLE). The respiratory disorders which DNase is thought to be useful in treating are characterised by an increase in mucus viscosity due to the presence of human and bacterial genomic DNA.
Mucus is a complex mixture of salts, water, glycoproteins, proteoglycans and proteins that cleanses, lubricates and protects many passageways in the body, including those in the lungs. However, the presence of genomic DNA and in particular long strands of genomic DNA, increases mucus viscosity to the point where mucociliary clearance is compromised. The viscous mucus is not cleared properly by the normal mechanisms and blocks or obstructs the airways of the lung, thereby restricting airflow. It may also promote the occurrence of infections. Overall the effect may be fatal or dehabilitating.
It is thought that the viscous genomic DNA in these respiratory conditions mainly originates from infiltrating inflammatory cells such as neutrophils. These cells are believed to undergo necrosis, rather than the more controlled process of apoptosis, releasing large amounts of gelatinous genomic DNA into the mucus. The release of other polymers, such as actin, from the necrosing cells may further increase the viscosity of the mucus.
Cystic fibrosis is one of the main respiratory disorders where abnormally increased mucus production and the presence of DNA in the mucus play an important role. Cystic fibrosis is an autosomal recessive inherited disorder caused by mutation of the Cystic Fibrosis Transmembrane Conductance Regulator (CFTR) gene. It is the most common inherited disorder in individuals of European descent and also occurs in other populations, but normally with a lower incidence. Patients with cystic fibrosis typically display difficulty in breathing, excessive salt loss during sweating, and incomplete digestion and absorption of food. The disease may also affect male fertility.
CFTR is a chloride channel and is present in mucus secreting cells. Chloride ions are released into the mucus via the CFTR channel. This raises the osmolarity of the mucus and results in the passage of water from the cells into the mucus, thinning it and helping to ensure that it has the correct viscosity. In cystic fibrosis patients, due to the defect in CFTR, chloride ions are not effectively transported into the mucus and hence the mucus is more viscous. The uptake of sodium, normally restricted by CTFR activity, is increased and, with it, water uptake leading to dehydrated condensed secretions that promote bacterial infection and hence inflammation. The number of white blood cells, and in particular neutrophils, in the mucus in the lung of cystic fibrosis patients is also greatly increased and necrosis of these cells releases genomic DNA which further increases mucus viscosity.
Cystic fibrosis is normally fatal, although life expectancy for afflicted individuals is increasing due to improved treatments. In the 1960's on average cystic fibrosis sufferers had a life expectancy of less than ten years, whereas now average life expectancy is approaching thirty years.
DNases, and in particular the recombinant human DNase I commonly used in the treatment of cystic fibrosis, are expensive. Furthermore, although DNases are presently used to treat cystic fibrosis patients, some 50 to 70% of cystic fibrosis patients show only minimal or no benefit following treatment.

### Summary of the Invention

The present invention is based on the finding that glycosaminoglycans which have an average molecular weight of from 8 to 40kd can increase the activity of DNase I. The glycosaminoglycan itself does not cleave DNA, rather it increases the ability of DNase I to do so. This unexpected synergistic effect means that less DNase I is necessary to achieve the same effect and also that higher levels of total activity may be achievable in a system using the same amount of DNase I.

The higher level of activity achievable with the same amount of enzyme may mean that conditions or individuals previously refractory to treatment with DNases, or which derived minimal benefit from treatment, may now be treatable. It may also mean that less DNase I has to be used which is in particular important in applications where recombinant DNases I are employed as these enzymes are expensive.

Accordingly, the present invention provides the use of a glycosaminoglycan or a physiologically acceptable salt thereof in the manufacture of a medicament for treating a subject with a disorder characterised by the presence of endogenous extracellular DNA, wherein the subject is also being treated with a DNase I, the glycosaminoglycan or salt thereof has an average molecular weight of from 8 to 40 kd and the glycosaminoglycan or salt thereof increases the activity of the DNase I, where the disorder is: (a) a respiratory disorder characterised by the presence of endogenous extracellular DNA in the lung; (b) selected from cystic fibrosis, chronic airflow limitation and pneumonia; (c) Systemic lupus erythematosus (SLE); or (d) a disorder characterised by the presence of endogenous extracellular DNA at an inflammatory site.

The present invention also provides the use of a DNase I in the manufacture of a medicament for treating a subject with a disorder characterised by the presence of endogenous extracellular DNA, wherein the subject is also being treated with a glycosaminoglycan or a physiologically acceptable salt thereof, the glycosaminoglycan or salt thereof has an average molecular weight of from 8 to 40kd and the glycosaminoglycan or salt thereof increases the activity of the DNase I, where the disorder is: (a) a respiratory disorder characterised by the presence of endogenous extracellular DNA in the lung; (b) selected from cystic fibrosis, chronic airflow limitation and pneumonia; (c) Systemic lupus erythematosus (SLE); or (d) a disorder characterised by the presence of endogenous extracellular DNA at an inflammatory site.

The present invention further provides a pharmaceutical composition comprising:

- a glycosaminoglycan or a physiologically acceptable salt thereof which has an average molecular weight of from 8 to 40kd;
- a DNAse I; and
- a pharmaceutically acceptable excipient, carrier or diluent.

The present invention also provides:

- products comprising a glycosaminoglycan or a physiologically acceptable salt thereof, the glycosaminoglycan or salt thereof having an average molecular weight of from 8 to 40kd, and a DNase I for use in the treatment of a disorder characterised by the presence of endogenous extracellular DNA in the subject to be treated, where the glycosaminoglycan or salt thereof increases the activity of the DNase I and wherein the products are to be administered in a simultaneous, separate or sequential manner, where the disorder is: (a) a respiratory disorder characterised by the presence of endogenous extracellular DNA in the lung; (b) selected from cystic fibrosis, chronic airflow limitation and pneumonia; (c) Systemic lupus erythematosus (SLE); or (d) a disorder characterised by the presence of endogenous extracellular DNA at an inflammatory site;
- an agent for use in treating a subject having a disorder characterised by the presence of endogenous extracellular DNA, the agent comprising a glycosaminoglycan or a physiologically acceptable salt thereof, the glycosaminoglycan or salt thereof having an average molecular weight of from 8 to 40kd, wherein the said subject is being treated with a DNase I and the glycosaminoglycan or salt thereof increases the activity of the DNase I, where the disorder is: (a) a respiratory disorder characterised by the presence of endogenous extracellular DNA in the lung; (b) selected from cystic fibrosis, chronic airflow limitation and pneumonia; (c) Systemic lupus erythematosus (SLE); or (d) a disorder characterised by the presence of endogenous extracellular DNA at an inflammatory site; and
- an agent for use in treating a subject having a disorder characterised by the presence of endogenous extracellular DNA, the agent comprising a DNase I, wherein the said subject is being treated with a glycosaminoglycan or a physiologically acceptable salt thereof, the glycosaminoglycan or salt thereof having an average molecular weight of from 8 to 40kd and the glycosaminoglycan or salt thereof increases the activity of the DNase I, where the disorder is: (a) a respiratory disorder characterised by the presence of endogenous extracellular DNA in the lung; (b) selected from cystic fibrosis, chronic airflow limitation and pneumonia; (c) Systemic lupus erythematosus (SLE); or (d) a disorder characterised by the presence of endogenous extracellular DNA at an inflammatory site.

Herein described is a kit comprising:

- a glycosaminoglycan or a physiologically acceptable salt thereof, the glycosaminoglycan or salt thereof having an average molecular weight of from 8 to 40kd;
- a DNase I; and
- packaging.

The invention also provides:

- a nebuliser or other liquid aerosol device, dry powder inhaler or metered dose inhaler comprising a composition according to the invention; and
- use of a glycosaminoglycan or a physiologically acceptable salt thereof, the glycosaminoglycan or salt having an average molecular weight of from 8 to 40kd to increase the activity of a DNase *in vitro.*

**Brief Description of the Figures**

Figure 1 shows the effect of unfractionated heparin on the degradation of DNA by DNase. The Y axis shows DNA concentration after incubation with DNase for 1 hour. The X axis shows the DNase concentration employed. Results are provided for incubations carried out in the presence of heparin (□) and in the absence of heparin (♦).

Figure 2 shows the effect of unfractionated heparin on the degradation of DNA by DNase as determined by agarose gel electrophoresis. The top panel shows the results of incubation of genomic DNA with DNase in the absence of heparin, whereas the bottom panel shows the results when the same digestions were performed in the presence of heparin. Lanes 1 to 12 are as follows: lanes 1 & 2 - DNA alone; lanes 3 & 4 - DNA + DNase (5 minute incubation); lanes 5 & 6 - DNA + DNase (15 minute incubation); lanes 7 & 8 - DNA + DNase (30 minute incubation); lanes 9 & 10 - DNA + DNase (45 minute incubation); lanes 11 & 12 - DNA + DNase (60 minute incubation).

Figure 3 shows the effect of unfractionated heparin on the degradation of DNA by DNase as determined by atomic force microscopy (AFM). Panel A shows the results for DNA incubated on its own. Panel B shows the results for DNA incubated with DNase. Panel C shows the results for DNA incubated with DNase and 1 microgram per ml of heparin. Panel D shows the results for DNA incubated with DNase and 10 micrograms per ml of heparin.

Figure 4 shows the effect of various glycosaminoglycans (GAGs) on DNase activity. The Y axis shows the concentration of DNA remaining after one hour incubation with DNase and the GAG, whilst the X axis shows the concentration of GAG employed. Results are shown for (A) heparin; (B) a mixture of chondroitin sulphates A and C; (C) heparan sulphate; (D) LMW heparin; and (E) dextran sulphate (which is a non-GAG control).

Figure 5 shows the effect of a mixture of chondroitin sulphates A and C on DNase efficacy in cystic fibrosis sputum as assessed using a barrier assay involving measurement of the transport of fluorescent microspheres. Results are shown for a variety of chondroitin sulphate concentrations.

Figure 6 shows the effect of a mixture of chondroitin sulphates A and C on the degradation of DNA by DNase as determined by atomic force microscopy (AFM). Panel A shows the results obtained in a DNA control with no DNase or chondroitin sulphate. Panel B shows the results for DNA incubated with DNase alone. Panel C shows the results for DNA incubated with both DNase and chondroitin sulphate.

**Brief Description of the Sequences**

SEQ ID NO: 1 provides the nucleotide and amino acid sequence for human DNase 1.

SEQ ID NO: 2 provides the amino acid sequence for human DNase 1.

**Detailed Description of the Invention**

The present invention is concerned with administration, to a subject having a disorder characterised by the presence of endogenous extracellular DNA, of a glycosaminoglycan or a physiologically acceptable salt thereof, to potentiate the activity of a DNase I that is administered to the subject to treat the disorder. The disorder is: (a) a respiratory disorder characterised by the presence of endogenous extracellular DNA in the lung; (b) selected from cystic fibrosis, chronic airflow limitation and pneumonia; (c) Systemic lupus erythematosus (SLE); or (d) a disorder characterised by the presence of endogenous extracellular DNA at an inflammatory site

The activity of the DNase I is thus enhanced. A synergistic effect can be observed. In an especially preferred

embodiment of the invention the glycosaminoglycan employed will be a heparin or a physiologically acceptable salt thereof. The DNase employed is a DNase I. In a further especially preferred embodiment the glycosaminoglycan will be administered intranasally and/or via inhalation.

*Synergy of glycosaminoglycan with DNAse I*

Unexpectedly glycosaminoglycans with an average molecular weight of from 8 to 40 kd are capable of increasing the activity of DNase I. That is that the same molar amount of enzyme has increased activity in the presence of glycosaminoglycan compared to in the absence of glycosaminoglycan. Glycosaminoglycan alone have no, or negligible, DNA hydrolytic activity thus the increase in activity is specifically due to an increase in the enzyme's activity in the presence of glycosaminoglycan.

The rate of DNA digestion i.e. the amount of DNA digested per unit time may be increased by from one, two, three, ten, twenty or more fold by the addition of the glycosaminoglycan. The enhancement in DNase I activity may typically be from 5% to 5000%, preferably from 50 to 2500%, more preferably from 75 to 1000%, still more preferably from 100 to 1000%, yet more preferably from 250 to 1000% and even more preferably from 500 to 1000%. These enhancements will typically refer to the amount of DNA the DNase I can degrade in a given time and preferably to the activity of the enzyme as expressed in Kunitz units or alternatively Dornase units.

One Kunitz unit of DNase I will produce a delta A260 of 0.001 per minute per ml at pH 5.0 at 25°C, using DNA as substrate, with [$Mg^{2+}$] = 4.2 mM. Preferably the DNA used to assess the DNAse I activity will be calf thymus or salmon sperm genomic DNA. A Dornase unit is defined as the amount of enzyme that will cause a decrease of 1.0 relative viscosity unit in a solution of highly polymerised DNA from the original viscosity of 4.0 in 10 minutes at 37°C.

The presence of glycosaminoglycan may effectively increase the number of units of DNase I enzyme activity present by more than one fold, double, treble, five fold, ten fold, twenty fold or more. Typically, the presence of glycosaminoglycan may effectively reduce the molar amount of DNase I necessary to achieve the same activity by a half, a quarter, a fifth, a tenth or more. The glycosaminoglycan may ensure a more complete digestion of DNA in a given time by the same amount of DNase I thus the average, or main fragment size, present may typically be twice, three, five, ten, twenty, fifty or more times longer in the absence than in the presence of glycosaminoglycan following incubation for an equivalent amount of time.

A number of methods are described herein for assaying for DNase I activity. Any of these may be used to assess DNase I activity. In particular a fluorescence based assay using, for example, Hoechst Stain may be used such as that described in Labarce & Paiden, 1980, Anal. Biochem., 102:344-352 to assay for DNA hydrolysis. Gel electrophoresis may be used to assess DNA fragment size.

The glycosaminoglycan or salt thereof is typically provided in an amount that produces a synergistic effect on DNase I activity. Thus one or more of the values given herein for increase in activity may be achieved. The ratio of DNase I to glycosaminoglycan, or salt by weight or alternatively by units may, for example, be from 1:50,000 to 1000:1, preferably from 1: 10,000 to 100:1, more preferably from 1: 5,000 to 50:1, still more preferably be from 1:1,000 to 25:1. The ratio may, for example, be from 1:500 to 1:20, preferably be from 1:150 to 1:5, more preferably be from 1:50 to 1:2 and even more preferably be from 1:10 to 1:1. The two may, for example, be in equal amounts or the DNase I may be two fold, five fold, ten fold or more excess or alternatively the glycosaminoglycan may be present in similar excess.

*DNAse 1*

Any suitable DNase I may be used in the present invention. The DNase is therefore a DNase I (EC 3.1.21.1). DNases I occurs in a number of species and any DNase I capable of cleaving DNA may be used in the invention. The DNase I may be from an animal source such as of bovine or porcine origin. It may be of plant, fungal, or microbial origin. However, typically and most preferably the DNase I is of human origin and is preferably a recombinant human DNase I. Commercially available DNase I preparations such as Dornase™ and Pulmozymele™ may be used in embodiments of the invention.

The DNase I will have DNA hydrolytic activity, for example it may hydrolyse DNA to give 5'-phosphate nucleotides. A fluorescence based assay using, for example, Hoechst Stain may be used such as that detected in Labarce & Paiden, 1980, Anal. Biochem., 102:344-352 to assay for DNA hydrolysis. Hydrolytic activity may be assessed in a variety of ways known in the art including analytical polyacrylamide and agarose gel electrophoresis, hyperchromicity assay (Kunitz, J. Gen. Physiol. 33:349-362 (1950); Kunitz, J. Gen. Physiol. 33:363-377 (1950)) or methyl green assay (Kumick, Arch. Biochem. 29:41-53 (1950); Sinicropi, et al., Anal. Biochem. 222:351-358 (1994)). The breakdown of DNA molecules from high to lower molecular weight forms may be monitored preferably by techniques such as agarose gel electrophoresis. Control experiments in the absence of the enzyme and/or in the presence of a protein known to possess DNase I activity may be performed.

The DNase I will preferably display mucolytic activity for samples of mucus containing DNA. Mucolytic activity

refers to the reduction of viscoelasticity (viscosity) of mucus. Mucolytic activity may be determined by any of several different methods known in the art, including sputum compaction assay (see for example WO 94/10567) assays using a torsion pendulum (Janmey, J. Biochem. Biophys. Methods 22:41-53 1991) or other suitable rheological methodologies.

DNase I is known to be prone to deamidation. Asparagine residues and in particular the asparagine at amino acid positions 7 and 74 of the mature human DNase I are prone to deamidation. This process converts the asparagine residues in question to aspartic acid or iso-aspartate residues. Deamidation reduces the activity of the enzyme and this is particularly the case for deamidation at the asparagine at amino acid position 74 of mature human DNase I.

Techniques are available for removing deamidated forms of the enzymes to leave the amidated forms and these may be employed to prepare DNase I for use in the invention. These techniques may include tentacle cation exchange (TCX) or affinity purification using DNA to purify the amidated forms of the enzyme which are still capable of binding DNA.

A DNase I preparation for use in the invention may typically comprise from 85 to 100%, preferably from 90 to 100%, more preferably from 95 to 100% and even more preferably from 99 to 100% amidated, or partially amidated, enzyme by weight. In particular these figures refer to the amount of wholly amidated enzyme i.e. with all of the residues which are naturally amidated being amidated. They will typically have more than 95%, preferably more than 99% and even more preferably more than 99.9% of the DNase I in an amidated form. In particular, these values refer to values at the time of production or to their values from one month to a year, preferably from two to six months and more preferably from three to four months after production. They may refer to the value during any point of the shelf-life of the product.

It is known that certain surfaces promote deamidation of DNase I and hence that the pharmaceutical compositions, preparations and medicaments of the invention are preferably not stored in contact with glass. They may, however, be stored in glass containers which are coated with a non-glass layer so that glass is not in contact with the DNase I. Alternatively they may be provided in plastic containers or other suitable non-glass containers.

Variants of naturally occurring or known DNase I may be used in the invention. Thus the term DNase I encompasses such variants. The term "variants" refers to polypeptides which have the same essential character or basic biological functionality as DNase I. The essential character of a DNase I is phosphodiesterase activity and the ability to hydrolyse DNA. Assays for measuring DNA cleavage are described herein and these may be used to determine whether a variant has hydrolytic activity.

SEQ ID NO: 2 provides the amino acid sequence for human DNase 1 and variants of this sequence are encompassed in the invention. Typically, polypeptides with more than 65% identity, preferably at least 80% or at least 90% and particularly preferably at least 95%, at least 97% or at least 99% identity, with the amino acid sequence of SEQ ID NO: 2 which retain DNA hydrolytic activity, are considered as variants which may be employed in the invention. Such variants may include allelic variants and the deletion, modification or addition of single amino acids or groups of amino acids within the protein sequence, as long as the peptide maintains DNA hydrolytic activity and more than 65% identity with the amino acid sequence of SEQ ID NO: 2.

Amino acid substitutions may be made, for example from 1, 2 or 3 to 10, 20 or 30 substitutions may be introduced. The modified polypeptide will retain DNase I activity. Conservative substitutions may be made, for example according to the following Table. Amino acids in the same block in the second column and preferably in the same line in the third column may be substituted for each other.

| | | |
|---|---|---|
| ALIPHATIC | Non-polar | G A P |
| | | I L V |
| | Polar-uncharged | C S T M |
| | | N Q |
| | Polar-charged | D E |
| | | K R |
| AROMATIC | | H F W Y |

Shorter variants are within the scope of the invention. For example, a peptide of at least 20 amino acids or up to 50, 60, 70, 80, 100, 150 or 200 amino acids in length is considered to fall within the scope of the invention as long as they demonstrate DNA hydrolytic activity and more than 65% identity with the amino acid sequence of SEQ ID NO: 2. In particular, but not exclusively, this aspect of the invention encompasses the situation when the protein is a fragment of the complete protein sequence of SEQ ID NO:2 and may represent a DNA-binding and hydrolytic region in particular include the active site.

A variety of programs may be used to calculate percentage homology. The UWGCG Package provides the

BESTFIT program which can be used to calculate homology (for example used on its default settings) (Devereux et al (1984) Nucleic Acids Research 12, p387-395). The PILEUP and BLAST algorithms can be used to calculate homology or line up sequences (typically on their default settings), for example as described in Altschul S. F. (1993) J Mol Evol 36:290-300; Altschul, S, F et al (1990) J Mol Biol 215:403-10.

Software for performing BLAST analyses is publicly available through the National Centre for Biotechnology Information (http://www.ncbi.nlm.nih.gov/). This algorithm involves first identifying high scoring sequence pair (HSPs) by identifying short words of length W in the query sequence that either match or satisfy some positive-valued threshold score T when aligned with a word of the same length in a database sequence. T is referred to as the neighbourhood word score threshold (Altschul *et al,* supra). These initial neighbourhood word hits act as seeds for initiating searches to find HSPs containing them. The word hits are extended in both directions along each sequence for as far as the cumulative alignment score can be increased. Extensions for the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLAST program uses as defaults a word length (W) of 11, the BLOSUM62 scoring matrix (see Henikoff and Henikoff (1992) Proc. Natl. Acad. Sci. USA 89: 10915-10919) alignments (B) of 50, expectation (E) of 10, M=5, N=4, and a comparison of both strands.

The BLAST algorithm performs a statistical analysis of the similarity between two sequences; see e.g., Karlin and Altschul (1993) Proc. Natl. Acad. Sci. USA 90: 5873-5787. One measure of similarity provided by the BLAST algorithm is the smallest sum probability (P(N)), which provides an indication of the probability by which a match between two nucleotide or amino acid sequences would occur by chance. For example, a sequence is considered similar to another sequence if the smallest sum probability in comparison of the first sequence to the second sequence is less than about 1, preferably less than about 0.1, more preferably less than about 0.01, and most preferably less than about 0.001.

The DNase I employed may actually be a variant which has reduced affinity for actin compared to the DNase I it is derived from. These variants may, for example, be used where actin is present in addition to the DNA and preferably where the disorder to be treated is a respiratory disorder where actin is present in the mucus.

The variant may have from 0 to 50%, preferably from 0 to 25 %, more preferably from 0 to 15 % and even more preferably from 0 to 5% of the affinity of the unmodified DNase I it is derived from for actin and preferably of that of human DNase I. The affinity of the variant for actin may be reduced from by from 1 to 1000 fold, preferably from 5 to 500 fold, more preferably by from 10 to 100 fold and even more preferably by from 20 to 50 fold. These figures may refer to affinity for polymeric or monomeric action or both. The variant may lack the ability to bind to specifically actin altogether. Variants with reduced activity for actin will, however, retain some, or all, of their hydrolytic ability and their ability to bind and cleave DNA.

Amino acid changes may be made to a DNase I to introduce a glycosylation site at, or near, to an actin binding site of the DNase I to reduce the affinity of the variant for actin. Regions or residues responsible for actin binding may be deleted or substituted with alternative amino acids. Insertions or modifications may be made close to actin binding sites to disrupt their function. Preferred DNase I variants with reduced affinity for actin are detailed in US Patent 6,348,343 and any of the DNase I variants detailed therein with reduced affinity for actin, but which retain DNA hydrolytic activity, may be employed in the present invention. The reduction in the ability to bind actin can then be determined to check affinity for actin is reduced, whilst DNA hydrolytic activity is retained.

The ability of a variant to bind actin may be monitored by techniques known in the art such as those detailed in Mannherz, et al., Eur. J. Biochem. 104:367-379 (1980). The relative binding affinities of different DNaseI may be determined by measuring the binding of the DNase I to immobilised actin in an ELISA assay or by comparing the DNA-hydrolytic activity of the DNase I in the presence and absence of actin.

The sequence of the DNase I may be modified so that it has extended half-life. For example the sequence of the enzyme may be changed to remove recognition sequences for certain proteases and in particular those derived from inflammatory cells present in the lung or other systems where the invention will be employed.

The DNase I may also be chemically modified to alter its properties such as, for example, its biological half-life. To achieve this covalent modifications may be introduced by reacting targeted amino acid residues of the native or variant.DNase I with an organic derivatising agent that is capable of reacting with selected amino acid side-chains or N- or C-terminal residues. Suitable derivatising agents and methods are well known in the art.

Residues which in particular may be derivatised include cysteinyl residues (most commonly by reaction with $\alpha$-haloacetates), histidyl residues (by reaction with diethylpyrocarbonate at pH 5.5-7.0), lysinyl and amino terminal residues (by reaction with succinic or other carboxylic acid anhydrides), arginyl residues (by reaction with reagents such as phenylglyoxal, 2,3-butanedione, 1,2-cyclohexanedione, and ninhydrin). Carboxyl side groups (aspartyl or glutamyl) may be selectively modified by reaction with carbodiimides or may be converted to asparaginyl and glutaminyl residues by reaction with ammonium ions. The covalent attachment of agents such as polyethylene glycol (PEG) or human serum

albumin to the DNases I may reduce their immunogenicity and/or toxicity of the variant and/or prolong its half-life and hence may be used in the invention.

The DNase I may be directly conjugated to the glycosaminoglycan or joined through an intermediate molecule.

*Glycosaminoglycans*

The medicaments and uses of the invention employ glycosaminoglycans as described in claims 1-21. Glycosaminoglycans are linear heteropolysaccharides possessing characteristic disaccharide repeat sequences that are typically highly N-and O-sulpated at D-glucosamine, galactosamine and uronic acid residues. These sulphate moieties introduce a high degree of negative charge along the glycosaminoglycan polymer chain and add to the heterogeneity of these macromolecules.

Any suitable glycosaminoglycan as claimed may be employed in the invention. Preferably, the glycosaminoglycan will be in a form suitable for administration intranasally, via inhalation, and/or via instillation as typically the medicaments of the invention will be administered by such routes. Glycosaminoglycans and glycosaminoglycan salts suitable for use in the present invention will have an average molecular weight of from 8 to 40 kd, preferably from 10 to 30 kd, more preferably from 12 to 20 kd. In particular, the glycosaminoglycan or salt may have an average molecular weight of from 14 to 18kd, preferably from 15 to 17 kd and more preferably from 16 to 17 kd. In some instances all, or substantially all, of the glycosaminoglycan molecules or glycosaminoglycan salt molecules will have a molecular weight falling within the ranges specified above. Thus from 50 to 100%, preferably from 75 to 100%, more preferably from 90 to 100%, still more preferably 95 to 100% of the molecules may have such a molecular weight. In some cases at least 95%, preferably 97.5%, more preferably 99%, still more preferably 99.5% and even more preferably 99.9% may have a molecular weight falling within the range. The glycosaminoglycan or salt herein described may be present in a range of molecular weight sizes and typically the most commonly occurring molecular weight size will fall within one of the above specified molecular weight ranges.

Preferably, the glycosaminoglycan employed in the invention will be any of chondroitin sulphates A to E, heparin, heparin sulfate, heparan, heparan sulphate, hyaluronic acid, keratan sulphate, or a mixture or any two thereof. Chondroitin sulphate B is sometimes referred to as dermatan sulphate. In a more preferred embodiment of the invention the glycosaminoglycan will be any of chondroitin sulphates A, C, D or E, heparin, heparin sulfate, heparan, heparan sulphate, hyaluronic acid, keratan sulphate, or a mixture of any two thereof. In a particularly preferred embodiment the glycosaminoglycan will be chondroitin sulphate A, chondroitin sulphate C, heparin, heparin sulfate, heparan, heparan sulphate, or a mixture of any two thereof. More preferably, the glycosaminoglycan will be chondroitin sulphate A, chondroitin sulphate C, heparin, or a mixture of any two thereof. In an even more preferred embodiment of the invention the glycoaminoglycan will be heparin. In some embodiments of the invention the glycosaminoglycan employed will be a mixture of more than two glycosaminoglycans from one of the above mentioned groups, such as a mixture of three, four or five of the glycosaminoglycans.

In embodiments of the invention where a mixture of two glycosaminoglycans is employed the two may, for example, be present in the ratio 1:1, 1:2, 1:4, 1:10 or 1:100. The ratio may be 90:10, 80:20, 70:30, or 60:40. Any suitable ratio may be employed and either glycosaminoglycan may be at the higher concentration. The ratio may be the same as the ratio in which the two are isolated when they are recovered from a common tissue using standard techniques. In a preferred embodiment of the invention a mixture of chondroitins A and C will be employed and in particular at a ratio of 80:20, preferably 75:25 and even more preferably 70:30 with chondroitin sulphate A being present at the higher level.

Typically, the glycosaminoglycan will not have been subjected to fragmentation to reduce its molecular weight. Usually, the glycosaminoglycan will not have been subjected to depolymerisation, such as by chemical or enzymatic means, to reduce its molecular weight. The average number of saccharide units in the polysaccharide chains of the glycosaminoglycan may typically be from 18 to 100, preferably from 30 to 80, more preferably from 40 to 60 and still more preferably from 5 to 60 units.

The glycosaminoglycan may be any suitable commercially available glycosaminoglycan and may, for example, be an unfractionated glycosaminoglycan. The glycosaminoglycan will have typically been isolated from a natural sources such as from an animal. In some cases, the glycosaminoglycan may have been synthesised rather than be a naturally occurring molecule.

In some cases the glycosaminoglycan may have been isolated from an animal, and in particular from animal tissues such as those of pigs or cattle. The glycosaminoglycan may have been obtained from tissues such as the lung, liver, or gut of an animal and in particular from beef lung or pork intestinal mucosa. The glycosaminoglycan may have been obtained from the skin of such an organism.

The glycosaminoglycan may have been isolated from a cartilagenous fish or other sea or freshwater organism. In some cases the glycosaminoglycan may have been isolated from a shark or squid and in particular from the cartilage of such an organism. The glycosaminoglycan may have been isolated from a sturgeon and in particular from a sturgeon notochord.

One of the specific glycosaminoglycans mentioned above may have been modified to generate a derivative of the glycosaminoglycan. Such derivatives may be used in the invention as long as they retain the ability to increase DNase I activity. Thus in the presence of the derivative a given molar amount of DNase I will display a higher level of activity than in the absence of the derivative. Thus in the case of heparin, the heparin may have been subjected to O-desulphation such as at least at the 2-O and 3-O positions. The same or equivalent modifications may be made to other glycosaminoglycans to generate derivatives for use in the present invention.

The glycosaminoglycan may have been subjected to acetylation, deacetylation, oxidation and/or decarboxylation such as, for example, periodate oxidation to generate a derivative. Heparinoids may be used in the invention.

Herein described is a glycosaminoglycan or a physiologically acceptable salt thereof comprising repeating disaccharide units of general formula (1):

-[A-B]- (1)

wherein:

each A is the same or different and represents a moiety of formula (i) or (ii)

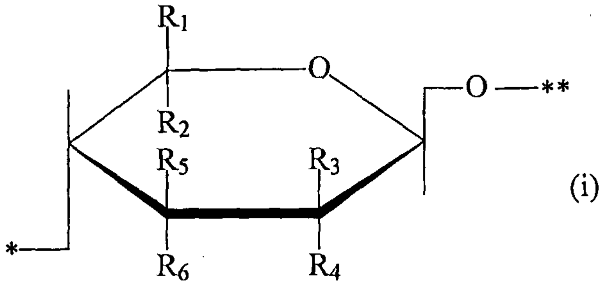
(i)
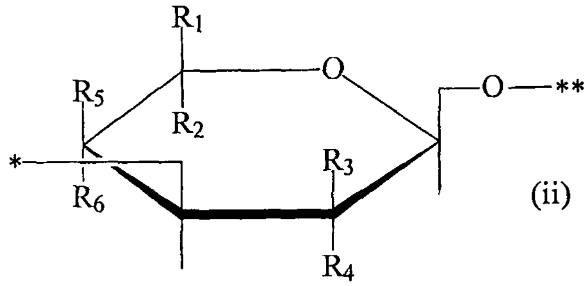
(ii)

wherein:

- one of $R_1$ and $R_2$ is hydrogen, and the other is $-CO_2H$, $-SO_3H$ or $-CH_2OR$ wherein R is hydrogen or $-SO_3H$;
- one of $R_3$ and $R_4$ is hydrogen, and the other is -OR wherein R is hydrogen or $-SO_3H$;
- one of $R_5$ and $R_6$ is hydrogen, and the other is -OH;
- * represents a direct bond to an adjacent hydrogen atom or B moiety; and
- ** represents a direct bond to an adjacent B moiety;

each B is the same or different and represents a moiety of formula (iii) or (iv);

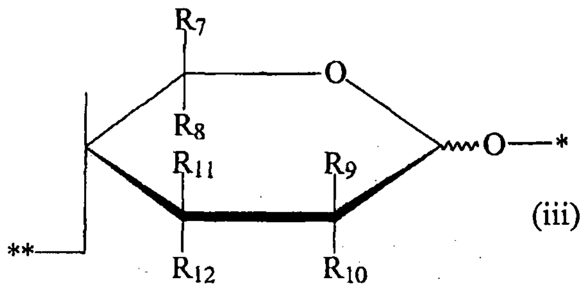
(iii)
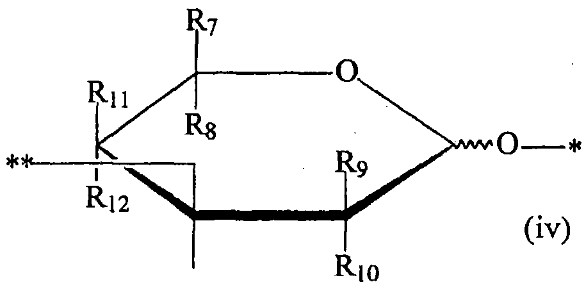
(iv)

wherein:

-one of $R_7$ and $R_8$ is hydrogen and the other is $-CH_2OH$ or $-CH_2OSO_3H$;
-one of $R_9$ and $R_{10}$ is hydrogen and the other is -NHAc, $-NH_2$ or $-NHSO_3H$;
-one of $R_{11}$ and $R_{12}$ is hydrogen and the other is -OH or $-OSO_3H$;
-* represents a direct bond to a hydrogen atom or an adjacent A moiety;
-** represents a direct bond to an adjacent A moiety; and
-indicates a bond in either stereochemical orientation;

or a physiologically acceptable salt thereof.

The formulae herein adopt standard practice in depicting sugars. According to this practice, the formulae include vertical lines through each of the cyclic carbon atoms. This does not, of course, mean that methyl groups are attached at each position, or that methylene groups are present as part of the link between adjacent cyclic moieties.

Preferably, each A moiety in the glycosaminoglycan of general formula (1) is the same. Preferably, each B moiety in the glycosaminoglycan of general formula (1) is the same.

Preferably, each A moiety in the glycosaminoglycan of general formula (1) is a moiety of general formula (i).

Typically, one of $R_1$ and $R_2$ is hydrogen, and the other represents $-CO_2H$ or $-CH_2OR$, wherein R is hydrogen or $-SO_3H$. Preferably, one of $R_1$ and $R_2$ is hydrogen and the other represents $-CO_2H$.

Typically, $R_3$ is hydrogen and $R_4$ is -OR, wherein R represents hydrogen or $-SO_3H$.

Typically $R_5$ is -OH and $R_6$ is hydrogen.

Typically each A is the same or different and represents a moiety of formula (i).

Typically $R_7$ is $-CH_2OH$ or $-CH_2OSO_3H$ and $R_8$ is hydrogen.

Typically $R_9$ is hydrogen and $R_{10}$ is -NHAc, $-NH_2$ or $-NHSO_3H$.

Typically $R_{11}$ is $-OSO_3H$ or -OH and $R_{12}$ is hydrogen.

Typically each B is the same or different and represents

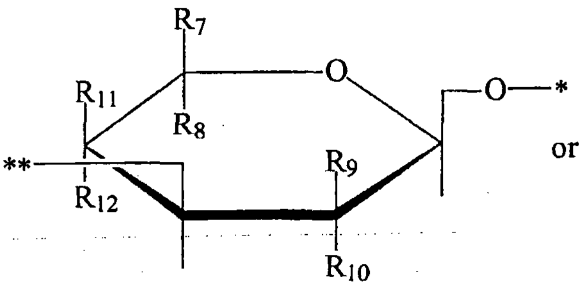

or

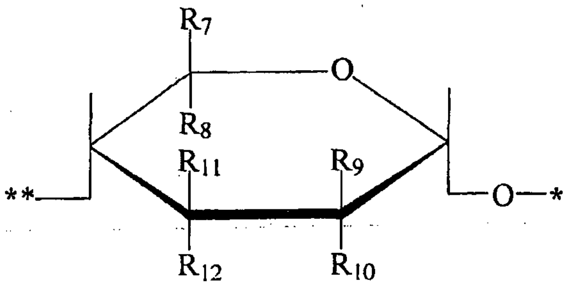

wherein $R_7$, $R_8$, $R_9$, $R_{10}$, $R_{11}$, $R_{12}$ * and ** are as described above.

Preferably $R_1$ is not hydrogen in an A moiety which is adjacent to a moiety (iv) in which $R_{12}$ is hydrogen.

Typically the glycosaminoglycan of general formula (1) is a glycosaminoglycan of general formula (2)

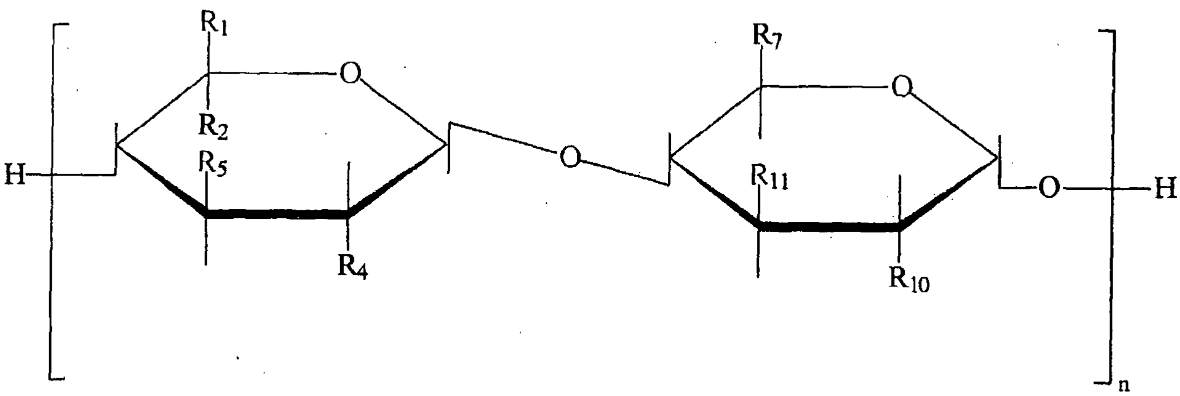

(2)

wherein:

- one of $R_1$ and $R_2$ is hydrogen and the other is $-CO_2H$;
- $R_4$ is -OH or $-OSO_3H$;
- $R_5$ is -OH;
- $R_7$ is $-CH_2OH$ or $-CH_2OSO_3H$;
- $R_{10}$ is $-NH_2$, $-NHSO_3H$ or -NHAc; and
- $R_{11}$ is $-OSO_3H$ or -OH.

Typically the glycosaminoglycan of general formula (1) is a glycosaminoglycan of general formula (3)

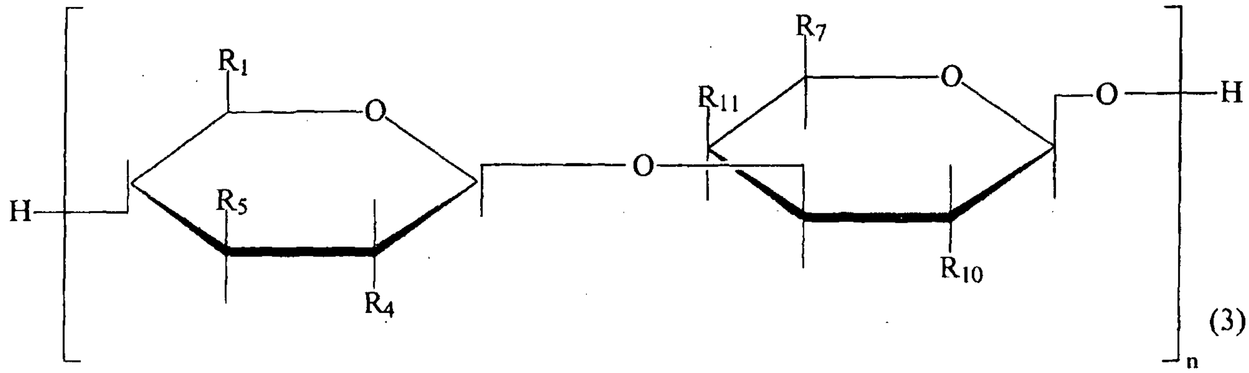

(3)

wherein:

- $R_1$ is $-CO_2H$;
- $R_4$ is -OH;
- $R_5$ is -OH;
- $R_7$ is $-CH_2OH$ or $-CH_2OSO_3H$;
- $R_{10}$ is -NHAc; and
- $R_{11}$ is -OH or $-OSO_3H$.

Any suitable physiologically acceptable glycosaminoglycan salt may be employed and in particular a metallic salt, for example a sodium salt, an alkali metal or an alkaline earth metal salt. Other salts include calcium, lithium and zinc salts. Ammonium salts may also be used. The salt may be a sodium glycosaminoglycanate or glycosaminoglycan sulphate. Salts of derivatives of specific glycosaminoglycans mentioned herein may also be used in the invention. In the present application where mention of a glycosaminoglycan is made, such mention also includes physiologically acceptable salts thereof.

Typically a physiologically acceptable salt is a salt with a physiologically acceptable acid or base. Preferred salts are salts with physiologically acceptable bases. Typically, such salts are compounds wherein the acidic hydrogen atom of a $-CO_2H$ and/or $-OSO_3H$ group is replaced with a cation, for example an alkali metal (e.g. sodium or potassium) or alkali earth metal (e.g. calcium or magnesium) cation. Such salts can be prepared, for example, by reaction with an appropriate hydroxide.

The number of disaccharide units present in the glycosaminoglycan or salt thereof employed in the invention will be such that the molecular weight of the glycosaminoglycan or salt is from 8 to 40 kd, preferably from 10 to 30 kd, more preferably from 12 to 20 kd. In particular, it may be such that the glycosaminoglycan has a molecular weight of from 14 to 18 kd, preferably from 15 to 17 kd and more preferably from 16 to 17 kd. The number of disaccharide units present in the glycosaminoglycan may be represented by the number n, where n is any integer such that the glycosaminoglycan has a molecular weight falling within any of the above mentioned molecular weight ranges.

Thus the glycosaminoglycan or salt employed may be represented by the general formula:

$$H\text{-}[A\text{-}B]_n\text{-}H$$

wherein -A-B is any of the disaccharides mentioned above and n is an integer such that the glycosaminoglycan or salt thereof has a molecular weight falling within the above specified molecular weight ranges. The value of n may be, for example, from 30 to 55 and more preferably from 35 to 50.

The glycosaminoglycan employed in the invention will typically comprise more than one length chain. Hence n for some of the glycosaminoglycan chains present may be a lower or higher integer than an integer which, on its own, would give a chain of molecular weight size falling within one of the above specified ranges. Thus the average value of n of the glycosaminoglycans present in the medicaments of the invention may be any of the values specified for n herein and in particular a value of n which gives a molecular weight glycosaminoglycan or salt falling within one of the molecular weight ranges specified herein.

Particularly preferred salts for use in the invention are salts of formula:

H-[A-B]nx-H Mx+

wherein x≤4n and M represents a physiologically acceptable cation or a mixture thereof. Most preferably, x≤2n.

In a particularly preferred embodiment of the invention the glycosaminoglycan employed will be a heparin or a physiologically acceptable salt thereof. Heparin is a naturally occurring mucopolysaccharide present in a variety of

organs and tissues, particularly liver, lung, and the large arteries. Heparin is a polymer of alternating $\alpha$-D-glucosamine and hexuronate residues joined by (1,4) glycosidic linkages. When glycosaminoglycans are synthesised in nature, typically they are conjugated to a central protein core. However, preferably the glycosaminoglycans herein described will lack such a central core. Typically, glycosaminoglycan preparations will lack a core and may be employed or, if present, the core can be removed. Commercially available preparations of glycosaminoglycans will usually lack the core and may be employed.

Heparin is clinically used as an anti-coagulant, where it is thought to exert its effects through interaction with anti-thrombin III (AT-III) and heparin co-factor II and other coagulation factors. Typically the heparin will retain some anticoagulant activity i.e. be able to increase clotting time in an individual. Thus preferably the heparin will be able to bind anti-thrombin III (AT-III) and/or heparin co-factor II (HCII) and hence inhibit clotting. Preferably it will be able to form a complex with AT-III, thrombin and a clotting factor. A heparin which lacks anti-coagulant activity or which has reduced anti-coagulant activity may also be employed. Thus the heparin may have been modified so that it has from 0 to 80%, preferably from 5 to 60%, more preferably from 10 to 40% and even more preferably from 10 to 30% of the activity of the unmodified form or in comparison to unmodified heparin. Other glycosaminoglycans, in particular dermatan sulphate, also possess anticoagulant activity. Preferably, therefore, the glycosaminoglycans and their derivatives employed will retain some anti-coagulant activity, as discussed above for heparin.

*Subjects to be treated*

The subject to be treated will have a disease or disorder characterised by the presence of endogenous extracellular DNA as claimed. That is there will be extracellular DNA present originating from the cells of the body of the subject. In particular, the disease or disorder is: (a) a respiratory disorder characterised by the presence of endogenous extracellular DNA in the lung; (b) selected from cystic fibrosis, chronic airflow limitation and pneumonia; (c) Systemic lupus erythematosus (SLE); or (d) a disorder characterised by the presence of endogenous extracellular DNA at an inflammatory site.

Typically the DNA will be present in one of the extracellular solutions or secretions of the body. Thus in preferred embodiments the DNA may be present in the lung including its airways. Typically this DNA will originate from the nucleus and hence be genomic DNA.

The extracellular DNA will typically be of high molecular weight. Thus it may be, for example, that it has an average fragment size, fragment size range or predominate fragment size in the range of from 100 bases to 1 megabase, preferably from 1 kb to 500 kb, more preferably from 5 kb to 250 kb in length, still more preferably from 25 kb to 100 kb and even more preferably from 25 kb to 50 kb in length. In embodiments of the invention where the condition to be treated is an autoimmune disorder such as SLE, the DNA may be of shorter length or may fall within the ranges specified above.

The DNA will typically be wholly, or partially free of, histones or will comprise regions which lack histones. It may be bound by cationic factors or factors with cationic groups such as inflammatory mediators and/or proteases. Thus for example it may be complexed or associated with elastase, cathepsins and/or IL-8.

The presence of the DNA will typically increase the viscosity of the solution or liquid it is present in. This will, in particular, be the case where the subject is suffering from a respiratory disorder where typically the presence of the DNA will increase the viscosity of mucus or other pulmonary secretions. It may also be the case where the object is to treat or help resolve an inflammatory site and in particular an enclosed inflammatory site in which there is extracellular DNA present.

Typically, the presence of DNA may increase the viscosity of the solution it is present in by from 10 to 5000%, preferably from 50 to 2500%, more preferably from 100 to 1000%, still more preferably from 200 to 800% and even more preferably from 400 to 600%. The viscosity of the solution may typically be doubled, tripled, quadrupled or increased by five fold or more in comparison to the viscosity of the solution in the absence of the DNA. Typically the viscosity of the DNA will be such that it may comprise such function in the subject such as, for example, lung function. It may cause airflow limitation and/or promote the occurrence of infections.

In one embodiment of the invention the subject may be suffering from inflammation and in particular inflammation where there is extracellular DNA at the inflammatory site. The main inflammatory site may, in particular, be enclosed either partially or totally. The site may contain or produce large amounts of pus or other inflammatory material. Examples of conditions include abscesses, meningitis, peritonitis, sinusitis, otitis, periodontitis, pericarditis, pancreatitis, cholelithiasis, endocarditis and septic arthritis. The subject may have inflammatory and infected lesions such as infected lesions of the skin and/or mucosal membranes, surgical wounds, ulcerative lesions and burns. The presence of the DNA may increase the viscosity of solutions at the inflammatory site and may slow down or prevent the resolution of the inflammation. It may also cause mechanical difficulties, thus for example, where the enclosed inflammatory site is a joint, or at a joint, its functioning may be compromised. Thus it may be harder to flex or manipulate. The subject may have an autoimmune disorder, such as one characterised by the production of an immune response against DNA and in particular antibodies

against DNA. In a preferred embodiment the subject may have, or be at risk of developing, systemic lupus erythematosus (SLE).

In a particularly preferred embodiment of the invention the subject will suffer from, a respiratory disorder and in particular one where the viscosity of mucus or other pulmonary secretions is elevated due to the presence of DNA. A subject herein described may, for example, have acute or chronic bronchial pulmonary disease, such as infectious pneumonia, bronchitis or tracheobronchitis, bronchiectasis, cystic fibrosis, asthma, tuberculosis, and/or fungal infections. The subject may have a respiratory tract infection. The subject may have sinusitis, sinus congestion or viral infections which infect the respiratory system such as a cold or flu. In a particularly preferred embodiment of the invention the subject will have cystic fibrosis. The subject may have pneumonia.

The disorder to be treated are those as described in claims 1-21 and will typically involve infiltration of inflammatory cells to the inflammatory site and in particular into the respiratory system. These cells may be macrophages, granulocytes, lymphocytes or other white blood cells. They may be T cells or B cells. The granulocytes may be eosinophils, basophils or neutrophils and in particular will be neutrophils.

The number of inflammatory cells present at the site, and in particular neutrophils, may typically be increased by from 1 to 10,000 fold, preferably, from 10 to 5,000 fold, more preferably from 20 to 1,000 fold, still more preferably by from 50 to 500 fold and even more preferably from 100 to 200 fold. The proportion and number of given white cell types may be determined by standard staining techniques well known in the art. Typically, many of the infiltrating inflammatory cells undergo necrosis or lysis to release genomic DNA into the mucus of the individual. Thus the number and/or proportion of necrotic cells may be elevated typically by from 10 to 5000 fold, more preferably by from 50 to 2000 fold, still more preferably from 100 to 1000 fold and most preferably from 200 to 600 fold. In particular the necrotic infiltrating cells primarily responsible for the release of genomic DNA into the mucus will be granulocytes and especially neutrophils.

The genomic DNA from the inflammatory cells may be predominately in the form of long strands of viscous genomic DNA. The necrosis of these cells may also be accompanied by the release of other proteins from the inflammatory cells such as inflammatory mediators and antibacterial proteins such as proteases like neutrophil elastase and/or cathepsins. As well as genomic DNA other polymers may also be present in the mucus further contributing to its viscosity. In many cases actin polymers may also be present further contributing to viscosity.

A contributing factor to the migration of inflammatory cells to the lungs may be the presence of pathogens, irritants, allergens or pollutants in the lung. For example, and in particular in cystic fibrosis, the subject may have infections such as *Pseudomonas, Pneumococcus, Staphylococcus, Burkholderia, Haemophilus* and/or *Aspergillus* infections and in particular may display infection with *Haemophilus influenzae, Staphylcoccus aureus, Pseudomonas aeruginosa,* and/or *Burkholderia cepacia.* These infections may be longstanding, occur periodically or be new. They may be acute or chronic. In some cases the pathogen will display antibiotic resistance due to prolonged exposure to antibiotics during treatment.

Subjects may also, or alternatively, have a history of exposure to pollutants such as tobacco smoke or allergens such as pollen and these may contribute to the influx of inflammatory cells into the lung. The presence of pathogen, allergens and/or pollutants may also promote the infiltrating cells to undergo necrosis rather than be disposed of via processes such as apoptosis where genomic DNA is not believed to be released into the lung in substantial amounts.

The subject may have a history of exposure to pollutants and/or chemicals and in particular to those in an inhaleable form. In particular, the subject may be a tobacco smoker or a former tobacco smoker. Typically, the subject may be, or have been, a heavy smoker, smoking from 10 to 100, preferably from 20 to 60, more preferably from 25 to 50 and even more preferably from 30 to 40 cigarettes per day. The subject may have done so for several years such as from 5 to 50, preferably from 10 to 40, more preferably from 15 to 30 and even more preferably from 20 to 25 years. The individual may have been, or be, a pipe or cigar smoker. The individual may have chewed tobacco or products containing tobacco. In some cases the individual may have been passively exposed to tobacco smoke rather than smoke tobacco themselves. Thus the subject may, for example, have been cumulatively exposed to passive tobacco smoke for long periods due to their work, leisure and/or home environments. The subject may use inhaled narcotic such as, for example, marijuana or other narcotics which are typically mixed with tobacco prior to smoking.

The subject may have additionally been, or alternatively be, exposed to other chemical or environmental pollutants. Thus the subject may work, or have worked, in an environment which exposes them to chemicals and/or pollutants. Thus, for example, the subject may be a factory worker or miner such a coal miner. The subject may work in the construction industry. The subject may have been exposed to high levels of pollution, such as exhaust emissions from vehicles or other engines. The subject may have been exposed to smog or sulphur dioxide containing emissions. The subject may have a genetic predisposition to developing a disorder and in particular a respiratory disorder.

In one of the preferred embodiments of the invention the subject will have chronic airflow limitation (CAL). In one embodiment of the invention the subject may have chronic airflow limitation (CAL). CAL is a disease state characterized by airflow limitation that is not fully reversible. The airflow limitation is usually both progressive and associated with an abnormal inflammatory response of the lungs to noxious particles or gases. In particular, although not exclusively,

CAL is associated with smoking.

CAL may be defined as a condition where there is a progressive decline in lung function, with the affected subject having an $FEV_1$ of less than 80% of that predicted for an individual of that age/race and/or height and/or who displays a $FEV_1$/FVC ratio of less than 70%. In an especially preferred embodiment, the subject to be treated with CAL will have an $FEV_1$ of less than 75% of that predicted. Typically the reduction of $FEV_1$ is only partially reversible. In particular the reduction in $FEV_1$ is only partially reversible by treatment with bronchodilators such as, for example, β2 adrenergic agonists and in particular salbutamol.

The $FEV_1$ for an individual with CAL may be from 10 to 80% of that predicted. Typically, the $FEV_1$ of the subject will be from 10 to 75% of the predicted value. Preferably the subject may have a $FEV_1$ of from 60 to 75% the predicted value, more preferably from 40 to 60% of predicted and even more preferably a value below 40% of that predicted. The subject with CAL may have an $FEV_1$ of less than 70%, preferably less than 60%, more preferably less than 50% and even more preferably less than 40% of that predicted. The $FEV_1$ value for the subject will normally be measured against predicted values and adjusted for age/sex/race and/or height. Predicted values may be those taken from Coates (*infra*). The expected value, which the value obtained for the subject with CAL may be compared to, may be the average expected value for smokers, or non-smokers, or both groups combined, preferably the expected value will be that for non-smokers not suffering from CAL (Coates, *infra*).

The reduction in $FEV_1$ in the subject with CAL will only be partially reversible and in particularly only be partially reversible on administration of a bronchodilator. Thus, for example, an increase in $FEV_1$ over the base-line value for the subject (i.e. that prior to administration of the bronchodilator) of more than 15%, preferably more than 20% and even more preferably over 25% will be regarded as reversibility. The increase may begin from 5 to 30, preferably from 10 to 25, more preferably over a period of from 15 to 20 minutes after the administration of the bronchodilator. Preferably the increases will begin from 15 minutes after the administration of the bronchodilator. The increases persist typically from 3 to 6 hours, preferably from 4 to 5 hours and more preferably 4 hours. Typically the bronchodilator used in assessing reversability will be a β2 adrenergic agonist such as salbutamol, or ipratropium. The reduction in $FEV_1$ may be totally, or almost totally refractory to treatment with bronchodilators.

The subject with CAL may also show similar minimal increases in $FEV_1$ with steroid drugs such as Becotide™ or Prednisolone™ although typically response to such agents will not be used to define reversibility. The increases will also occur over a longer time period such as after 2 to 3 days and, if the steroid drugs are continually administered, persist. If steroids are stopped the improvement may persist for from 12 to 48 hours, or for days, weeks or even months, such as from six hours to six weeks, preferably from 1 day to 3 weeks.

Tests to assess reversibility of reduction of $FEV_1$ will typically be performed when the subject is clinically stable and free from infection. The subject should not have taken, or have had administered to them, inhaled short-acting bronchodilators in the previous six hours, long-acting β agonists in the previous 12 hours or sustained release theophyllines in the preceding 24 hours.

In the diagnosis of CAL spirometric values should typically be measured before and after an adequate dose of inhaled bronchodilator is given to the subject. The dose should preferably be selected to be high on the dose/response curve and usually will be given by nebuliser to be certain it has been inhaled. A similar dose may be given with multiple inhalations from a metered dose inhaler and large volume spacer, but this is less preferred. A typical dosage/measurements protocol for a human subject would be:

- before and 15 minutes after 2.5 to 5mg nebulised salbutamol or 5 to 10 mg terbutaline;
- before and 30 minutes after 500 μg nebulised ipratropium bromide; or
- before and 30 minutes after both in combination.

Equivalent protocols may be used for non-human subjects.

The FVC of the subject may also be measured in the diagnosis of CAL. The ratio of $FEV_1$ to FVC can be used in the diagnosis of CAL. Subjects to be treated who have CAL will typically have an $FEV_1$ /FVC value of less than 70%. The ratio of $FEV_1$/FVC may be below 65%, preferably below 60%, more preferably below 55% and even more preferably below 55%. A subject herein described may have a $FEV_1$/FVC of below 70% and also have a $FEV_1$ value of 80% or less of that predicted.

FVC (forced vital capacity) corresponds to the maximal volume of air forcibly exhaled from the point of maximal inhalation and can be measured using standard spirometry. In particular, the above specified values for $FEV_1$ /FVC will be those after administration of a bronchodilator as outlined above. The reduction in $FEV_1$/FVC will typically show the same lack of reversibility as $FEV_1$ in subjects with CAL.

Spirometric assessment is the most preferred method for diagnosing CAL and hence method for identification of subjects who may be treated. Accordingly, in an preferred embodiment of the invention spirometric assessment will be used in the diagnosis of a subject who has CAL and hence is treatable using the invention. In addition, the symptoms displayed by the subject may also be assessed to help confirm a diagnosis of CAL. Typically diagnosis will involve

spirometric assessment in combination with assessment of the symptoms of a subject as well as elucidating whether the subject has a history of exposure to risk factors. In some situations spirometric assessment may not be possible, particularly in situations where resources are limited, and CAL will be diagnosed by alternative means such as by looking for the symptoms of CAL listed herein and a history of exposure to risk factors for CAL. Although chest X-rays are not typically indicative of whether or not a subject has CAL, they may be used to diagnose other respiratory disorders, such as TB, and hence rule out CAL.

The subject with CAL will show, or have previously shown, an accelerated rate of decline of lung function compared to the average expected for an equivalent individual not suffering from CAL and in particular for an equivalent non-smoking individual. The subject may display shortness of breath and in particular may do so after physical exertion such as on exercise. Typically, this will not be induced by exposure to an allergen. The subject may also show increased incidence of bacterial or viral infection and this may exacerbate the condition.

The individual with CAL may show a rate of decrease in $FEV_1$ one, two, three, four or more times greater than the average annual value expected for an equivalent individual not suffering from CAL. For instance a subject over thirty may show an annual reduction of from 50 to 100, preferably from 50 to 80 and more preferably from 60 to 70 ml $FEV_1$/yr compared to a reduction of from 10 to 40 and typically of 20 to 40 ml of $FEV_1$/yr in the equivalent non-smoker. These values may also apply to non smoking sufferers of CAL such as where the disorder is caused by pollutants.

Subjects with CAL may display one or more, and sometimes all, of cough, increased sputum production, dyspnea, and/or a history of exposure to risk factors for the disease. In the case of cough, increased sputum and dyspnea these may have been present for extended periods of time such as at least a month, preferably six months, more preferably at least a year and still more preferably for at least two years. Chronic cough and sputum production often precede the development of CAL and may be indicative of individuals for which the invention can be used prophylactically to prevent the development of CAL.

Subjects with CAL may have a history of exposure to pollutants, including any of those mentioned herein and at any of the levels specified herein. In particular, the subject with CAL will have a history of exposure to tobacco and/or industrial pollutants.

The subject with CAL will typically be a mature adult. For example, the subject may be from 21 to 85, preferably from 25 to 70, more preferably from 30 to 60 and even more preferably from 40 to 50 years of age. The onset of any of, or a particular, symptom mentioned herein in association with CAL, will typically have been in adulthood. For example, the subject may have been at least 20, more preferably at least 25, still more preferably at least 30 and even more preferably at least 35 years of age before they experienced a particular symptom. In particular, the symptoms associated with more advanced stages of CAL, such as any of those mentioned herein, may have their onset at such later stages of life. Subjects with a genetic predisposition to developing CAL, such as those with $\alpha_1$-antitrypsin deficiency, may develop CAL earlier. For example, they may display one or more, or a particular, symptom at from 10 to 21, preferably from 12 to 18, or more preferably from 14 to 16 years of age. Alternatively, they may first show the symptom at any of the age ranges mentioned herein. The subject may have been diagnosed at any of the ages, or within any of the age ranges, specified herein. These ranges may also apply to any disorder which the subject may have and in particular those mentioned herein.

The subject may have a genetic predisposition to developing CAL and may display a family history of the condition. For example, the subject may have $\alpha_1$-antitrypsin deficiency and hence be predisposed to developing CAL. Subjects at risk of developing CAL may have had low birth weights and/or a history of exposure to pollutants in the womb or in early life. The mother of the subject may be a smoker and may have continued to smoke during pregnancy. The subject may have had a history of severe childhood respiratory infection. Reduced maximal attained lung function, as measured by spirometry, may identify individuals who are at increased risk of developing CAL.

The subject may have an early stage of CAL in which the symptoms are generally moderate and may have periods of normality or of at least reduced symptoms. Alternatively, the subject may have a more developed stage of CAL in which the symptoms, and particular the reduction in $FEV_1$ is more pronounced. Preferably, the methods and medicaments of the invention are used, or administered at, an early stage of CAL so that they can arrest, slow or regress the increased rate in $FEV_1$ decline at as early a stage as possible.

CAL is typically a progressive disorder with the severity of CAL and the extent to which it has an impact on the sufferer increasing over time. Thus there may be a progressive manifestation in the symptoms of the disorder. Chronic cough is usually the first symptom to develop. It may initially be intermittent, but later may be present every day. The cough will typically be present throughout the day, rather than just at night and in the morning. In some cases, significant airflow limitation may develop without the presence of a cough. Small amounts of tenacious sputum are commonly raised after coughing bouts.

As CAL progresses the subject may experience dyspnea. The onset of dyspnea will often be one of the reasons why a human subject will first consult a physician as it can be dehabilitating and also induce anxiety. As the lung function of the subject deteriorates further, breathlessness becomes more intrusive. The subject may display wheezing and chest tightness. The dyspnea may become worse during or after exercise. Respiratory infections may also exacerbate the

condition and cause increased dyspnea. Human subjects may indicate that the dyspnea progressively impairs their ability to carry out physical labour and to exert themselves.

CAL sufferers are sometimes split into categories which reflect the stage and severity of the disease. This can help define the needs of a particular subject and what course of treatment should be administered. In one classification (GOLD Executive Summary, supra) subjects are split into the following categories:

Category 0 - At risk

Lung function, as measured by spirometry, is normal.

Chronic symptoms (cough, sputum, production).

Typically, human subjects will be unaware of abnormal lung function.

Category I: Mild CAL

Mild airflow limitation.

$$FEV_1/FVC < 70\%.$$

$FEV_1$ greater than, or equal to, 80% of that predicted.

With or without chronic symptoms (cough, sputum, production).

Human subjects may be unaware that their lung function is abnormal.

Category II: Moderate CAL

Worsening airflow limitation.

$$FEV_1/FVC < 70\%.$$

$FEV_1$ from 30 to 80% of that predicted (category can be subdivided into: IIA - $FEV_1$ from 50 to 80% of that predicted; and IIB - $FEV_1$ from 30 to 50% of that predicted). With, or without, chronic symptoms (cough, sputum, production, dyspnea). The symptoms may typically become more pronounced on exertion. Human subjects are likely to be aware of the condition and have sort medical advice.

Category III: Severe CAL

Severe airflow limitation.

$$FEV_1/FVC < 70\%$$

$FEV_1$ less than 30% predicted or alternatively an $FEV_1$ less than 50% of that predicted in conjunction with respiratory failure or clinical signs of right heart failure (respiratory failure: arterial partial pressure of oxygen ($PaO_2$) less than 8.0 kPa (60 mm Hg) with or without arterial partial pressure of $CO_2$ ($PaCO_2$) greater than 6.7 kPa (50 mm Hg) while breathing air at sea level).

Subject to be treated using the invention who have CAL may fall within any of the above categories. In particular, the subject may be one in categories I to III, preferably in categories II to III, and even more preferably in category III. The invention also encompasses the treatment of individuals at risk of CAL (category 0) and with an early stage of the

disorder (category I).

Some of the symptoms of CAL are displayed by sufferers of other diseases. For example, a number of respiratory disorders other than CAL compromise lung function. However, these diseases may be distinguished from CAL by a full assessment of the subject and preferably by applying the relevant available guidelines for the diagnosis of CAL. The British Thoracic Society have published a set of Guidelines (Thorax 1997, 52 (Suppl. 5): S1-28) and in a preferred embodiment a subject to be treated will fall within the definition of the disorder provided by these Guidelines. In addition, the Global initiative for chronic Obstructive Lung Disease (GOLD) has published an Executive Summary (2001) outlining a strategy for the diagnosis, management and prevention of the disorder. The subjects to be treated who have CAL will fall within the definition of the disorder provided by the Executive Summary.

CAL does not include cystic fibrosis (CF), although the invention can be used to treat both CAL and CF. Typically a subject with CAL will have a least one functional copy of the CFTR gene (unless they have been unfortunate enough to develop both CF and CAL). CAL typically has a much later onset than Cystic Fibrosis, where a sufferer is born with the defect. In CAL the decline in lung function and the onset of symptoms will occur progressively over time. In CF the decline in lung function will have a more immediate onset, earlier on in life. A subject who has CAL will often be in their thirties, forties or even fifties before they become aware that they are suffering from the disorder. The major exception to the later onset of CAL, is in those sufferers who have $\alpha_1$ anti-trypsin deficiency. Such subjects will display an earlier onset of CAL, such as in their teens or twenties when CAL is diagnosed, as they will be born with a genetic condition predisposing them to CAL. Subjects who have $\alpha_1$ antitrypsin deficiency and CF can readily be distinguished from each other by biochemical and genetic tests to identify the nature of the disorder.

The subject to be treated will be a vertebrate animal and preferably will be a mammal. Typically the subject will be human. However, the invention also encompasses the treatment of animals with conditions which are the same as, or equivalent to, any of the human conditions mentioned herein. Thus the animal condition may have either an underlying pathology similar to, or the same as, that of the human condition. The animal may have one or more similar symptoms or characteristics of the human condition and in particular one or more of those listed above. Preferably, the animal will suffer from a condition which falls within the definition of any of these conditions listed above.

In cases where the subject is not human it may be a domestic animal or an agriculturally important animal. The animal may, for example, be a sheep, pig, cow, bull, poultry bird or other commercially farmed animal. In particular the animal may be a cow or bull and preferably is a dairy cow. The animal may be a domestic pet such as a dog, cat, bird, or rodent. The animal may be a cat or other feline animal. The animal may be a monkey such as a non-human primate. For example, the primate may be a chimpanzee, gorilla, or orangutan. The animal may be a horse and, for example, may be a racehorse. The animal may be a sports animal such as, for example, a greyhound.

*Subject assessment*

The present invention provides medicaments, and preparations for the hydrolysis of DNA for use in the treatment of conditions caused by, or characterized by, the presence of extracellular endogenous DNA as defined in claims 1-21. One of the preferred ways of assessing the efficacy of various embodiments of the invention is in determining the amount of DNA present following treatment. Preferably, the change in the amount of DNA and in the range of DNA fragment sizes will typically be determined following administration of the medicaments of the invention compared to the situation prior to their administration. Any of the methods for measuring the amount, and fragment size, of DNA discussed herein may be utilised. The range of DNA fragment sizes may be determined by gel electrophoresis or other separation techniques based on separation of DNA by size.

The viscosity of the solution the DNA is present in, and in particular mucus viscosity, may be determined. Again, preferably the situation is compared prior to and post treatment. Any of the techniques discussed herein for measuring viscosity may be used such as, for example, techniques like compaction assays and assays using contortion pendulums or any other suitable rheological methods.

The medicaments herein described may reduce the viscosity of the DNA containing solution by from 0 to 100%, preferably from 20 to 100%, more preferably from 50 to 100% and even more preferably from 75 to 100% of that prior to administration of the medicament. Preferably, the average length of DNA fragment present or the predominate fragment size will be reduced by from 1 to 1000 fold, more preferably by from 5 to 500 fold, more preferably by from 10 to 100 fold and still more preferably by from 20 to 50 fold. The average DNA fragment size, or the predominate fragment size, may typically be from 50 bp to 10 kb, preferably from 100 bp to 2,500 bp, more preferably from 250 bp to 1,000 bp and even more preferably from 400 bp to 600 bp following treatment. The treatment may result in a visible shift towards lower molecular weight DNA fragments as visualised, for example, by gel electrophoresis and ethidium bromide staining and in particular may reduce the proportion of DNA species on the gel above 2 kb, preferably above 5 kb, more preferably above 10 kb and still more preferably those above 20kb on the gel.

The above and below mentioned changes may be observed within hours or days of administration, such as from one hour to seven days, preferably from two hours to two days and more preferably from four hours to 24 hours

after administration.

The assays to assess viscosity and DNA may also be performed on samples taken from the subject to assess the optimal DNase I and glycosaminoglycan concentrations and various other factors to use. Thus by performing experiments in *vitro* on samples such as sputum to determine how to optimally hydrolyse DNA, the medicament to be administered to the subject may be tailored to their needs. It may be possible to do this so that at times of particular severity of the disorder or particular circumstances the treatment regimen can be adjusted appropriately.

The administration of the medicaments may typically produce an improvement in the status of the subject. For example, in the case of respiratory disorders administration may result in a reduction of airway obstruction, mucus plugging, foaming and/or bubbling. The subject may show an increase in $FEV_1$ returning it to a value closer to that predicted for an equivalent subject not suffering from respiratory disorder.

In some cases, on treatment the subject will display an improvement of $FEV_1$ so that $FEV_1$ increases by from 25 to 100%, preferably by from 40 to 100%, more preferably from 60 to 100% and even more preferably from 80 to 100% of the predicted value. The improvement may also be smaller, such as from 5 to 10%, preferably from 15 to 25%, or more preferably from 20 to 25%. This may be accompanied by the subject experiencing increased ease in breathing, promoted expiration of mucus and reduced coughing. The subject may show increased ability to perform exercise and physically exert themselves.

The mucolytic effect of the DNase I may also decrease the incidence of respiratory infections, in particular where the subject has cystic fibrosis or pneumonia. Thus the subject may show a decrease in infections with pathogens such as, for example *Pseudomonas, Pneumococcus, Staphylococcus, Burkholderia, Haemophilus* and/or *Aspergillus.* In particular, the patient may show reduced incidence of infection with *Haemophilus influenza, Staphylococcus aureus, Pseudomonas aeuroginosa* and/or *Burkholderia cepacia.* Subjects where such infections were chronic may no longer be infected or have decreased load of pathogen following treatment. Pathogenic load and the nature of pathogens can be assessed using various microbiological techniques well known in the art such as staining, culturing and plating

In cases where the subject is suffering from an autoimmune disease characterised by the presence of antibodies reactive against DNA the efficacy of the invention may be assessed by monitoring for a decrease in the immune response against DNA in the subject. Thus typically the titre of antibodies against DNA present in the blood will be measured as may be the affinity of the antibodies present. The titre of the antibodies or their affinity may be decreased, for example, by a factor of two, four, five, ten, twenty, fifty or more fold. It may be decreased by more than 20%, preferably more than 40%, more preferably more than 60% and even more preferably by more than 90%. Assessment of antibody titre and affinity may be done using standard techniques such as ELISA assays. The severity of the disorder in the patient may be monitored.

The medicaments of the invention will typically treat the disorder in question. They may ameliorate its severity and/or eliminate or reduce symptoms associated with the disorder. In particular they will reduce or eliminate characteristics of the disorder associated with the presence of extracellular DNA. Thus in respiratory disorders such as cystic fibrosis they will have a mucolytic effect reducing mucus viscosity. This will help in the clearance of the buildup of mucus and there may be increased expectoration of mucus. There may be an improvement in lung function and a decreased incidence, or severity, of infection. The subject may have increased sense of well being. In some cases on treatment the subject will display an improvement of $FEV_1$ so that $FEV_1$ is from 25 to 100%, preferably from 40 to 100%, more preferably from 60 to 100% and even more preferably from 80 to 100% of the predicted value.

$FEV_1$ is defined as the maximal forced volume which can be expired in one second starting from maximum inspiration (European Resp. Journal, 1993; 6: Suppl. 16 and Coates, Lung Function,: Assessment and Applications In Medicine, 4th Edition, Oxford, Blackwell, 1969). It can be measured by standard techniques well known in the art i.e. by spirometry.

The medicaments may increase life expectancy such as by, for example, 1 to 2 years, preferably from 2 to 4 years and even more preferably by more than 4 years.

*Products*

The present invention also provides products comprising a glycosaminoglycan or a physiologically acceptable salt thereof, the glycosaminoglycan or salt thereof having an average molecular weight of from 8 to 40kd, and a DNase I for use in the treatment of a disorder characterised by the presence of endogenous extracellular DNA in the subject to be treated, where the glycosaminoglycan or salt thereof increases the activity of the DNase I and wherein the products are to be administered in a simultaneous, separate or sequential manner, where the disorder is: (a) a respiratory disorder characterised by the presence of endogenous extracellular DNA in the lung; (b) selected from cystic fibrosis, chronic airflow limitation and pneumonia; (c) Systemic lupus erythematosus (SLE); or (d) a disorder characterised by the presence of endogenous extracellular DNA at an inflammatory site.

These may typically be in a solid or liquid form. It may be in the form of a powder. It may be lyophilised or frozen. It may also comprise other components such as water or a buffer. The product may take the form of an aqueous

solution comprising both the DNase I and the glycosaminoglycan or salt. The product may also comprise stabilising agents or preservatives. It may comprise agents which allow the product to be frozen without losing enzyme activity such as glycerol. The products may be formed, for example by mixing powders of glycosaminoglycan and DNase.

*Administration and pharmaceutical compositions*

The glycosaminoglycan or physiologically acceptable salt may be administered simultaneously, separately or sequentially to the DNase I. Thus the two may be administered in the same medicament or as two separate medicaments and may be given at the same time, one after the other or separately. In embodiments where the two are to be administered separately, preferably the glycosaminoglycan, or salt will be administered prior to, or at the same time, as the DNase I.

The glycosaminoglycan and DNase I may typically be administered from one week to one minute apart, preferably from two days to one hour apart, even more preferably from 24 hours to 2 hours apart and still more preferably from 12 to 4 hours apart. The two may be administered from one minute to one hour apart, preferably from 10 minutes to 30 minutes apart and even more preferably from 15 to 25 minutes apart. The two may be administered from one minute to ten minutes apart, preferably from two minutes to eight minutes apart and more preferably from four to six minutes apart. In some cases they may be administered in succession, one directly after the other.

The two may be administered by the same route or via different routes, preferably the two will be administered by the same route either as separate medicaments or in the same medicament. The glycosaminoglycan or salt and the DNase I may be provided in two separate compositions, but may, for example, be mixed prior to addition to a delivery device or in the delivery device. The delivery device may have means to regulate the amount of each delivered to the subject. It may have means to mix the two or deliver each separately.

The medicaments and compositions described herein may be prepared by formulating the active agents, i.e the glycosaminoglycan or salt and/or the DNase I, with a standard pharmaceutically acceptable carrier and/or excipient as is routine in the pharmaceutical art. The exact nature of the formulation will depend upon several factors including the particular glycosaminoglycan, salt or DNase I employed and the desired route of administration. Suitable types of formulation are fully described in Remington's Pharmaceutical Sciences, 19th Edition, Mack Publishing Company, Eastern Pennsylvania, USA. The necessary dose to be administered will normally be determined by a physician, but for the glycosaminoglycan, or salt will, for example, be from 0.01mg to 5g, preferably from 0.1 mg to 2.5g, more preferably from 1 mg to 1g, even more preferably from 10 mg to 500mg , still more preferably from 50 mg to 250 mg and even more preferably from 100 mg to 200 mg. These doses will typically be given once, twice or three times a day and will preferably be given once or twice a day and more preferably will be given twice a day.

If the glycosaminoglycan is heparin or a physiologically acceptable salt thereof the dosage administered may be typically be in the range of from 10 to 10,000 units per/kg body weight, preferably 100 to 10,000 units per/kg body weight, more preferably from 200 to 5000 units/kg body weight, even more preferably from 500 to 2000 units per/kg, and still more preferably from 1,000 to 1,500 units per/kg.

Typically the dose of DNase I will be such that the concentration of DNase I achieved at the target site is from 100 to 0.001 μg/ml, preferably from 50 to 0.1 μg/ml, still more preferably be from 25 to 0.5μg/ml, yet more preferably be from 10 to 1 μg/ml. The concentration achieved at the target site may be from 8 to 2 μg/ml and preferably from 4 to 2 μg/ml. These doses will typically be given once, twice or three times a day and will preferably be given twice a day.

The length of treatment may typically be from a day, one week, two weeks, a month, six months a year or more. In many cases the subject will remain on the medicaments of the invention permanently or for extended periods. This may in particular be the case where the subject has a genetic defect, such as where the subject has cystic fibrosis, and hence permanently has the condition. This may also be the case where one of the causative factors in the disorder is something the subject is continuously exposed to and cannot avoid, or does not wish to minimise exposure to. The subject may be a tobacco smoker, but not give up smoking tobacco.

In cases where the disorder is more short lived, or occurs periodically, the length of administration may be shorter and may be linked to the duration of the disorder. Thus, for example, where the condition is an abscess or other inflammatory situation such as one involving an enclosed inflammatory site, once the inflammation has been resolved the administration of the medicament may be ceased or reduced. The administration schedule may be coordinated so that higher levels of the medicaments are administered, or initiation of administration begins, at times when the disorder has increased severity. Thus in respiratory disorders such as, for example, cystic fibrosis or pneumonia this may be when the subject has an increase in airway obstruction, infection and/or inflammation. The medicament may also be given prior to exercise or other physical exertion.

In cases where the disorder involves a specific inflammatory site the medicament may be administered directly to the inflammatory site or in the region of the site. Thus the medicaments may, for example, be injected into the site, applied as a cream or on the arm *etc.* The medicament may be administered via an implant. Thus herein described is an implant comprising a medicament of the invention. In auto-immune disorders the medicaments may typically be systemically administered such as by intravenous injection. In particular this may be the case for SLE.

Preferably the medicaments of the invention may be administered via inhalation and/or intra-nasally. Thus they may be delivered via the nose and/or mouth. Suitable methods for formulating and preparing medicaments to be administered via inhalation, instillation and intranasally are well known in the art. The medicaments may also be administered via instillation. In a preferred embodiment, the medicaments of the invention are suitable for administration by inhalation. For inhalation therapy the medicament may be in a solution useful for administration by liquid aerosol, metered dose inhalers, or in a form suitable for a dry powder inhaler. The medicament may be present in a blister pack or breakable capsule.

In some cases the medicaments may be administered via instillation. In such cases, typically the medicament will be in liquid form and will be administered via an artificial airway such as, for example, an endotracheal tube. The liquid will typically be drawn up into a syringe and then expelled through the artificial airway into the respiratory tract of the subject. Instillation is often used in an emergency context. In many cases it may be used where the subject has a relatively advanced form of CAL and has been admitted to hospital. Typically volumes such as from 1 to 20 ml, preferably from 2 to 10 ml, and even more preferably from 3 to 6 ml will be instilled. Any method for delivery into the pulmonary tract may be used to deliver the medicaments to the invention.

In cases where the medicament is to be administered via the nose it may be, for example, in the form of nasal spray. The spray may be administered, for example, using an atomizer or nebulizer. In some cases the medicament may be in the form of nasal drop. These may be administered using a via a medicine dropper. For further discussion on nasal dosage forms see Remington's Pharmaceutical Sciences (*Supra*). Medicaments administered via the nose may include pH adjusters, emulsifiers or dispersing agents, preservatives, surfactants, gelling agents, or buffering agents as may the other medicaments of the invention. Most preferably the nasal dosage form will be isotonic with nasal secretions.

The medicaments herein described may be formulated as aerosols. The formulation of pharmaceutical aerosols is routine to those skilled in the art, see for example, Sciarra, J. in Remington's Pharmaceutical Sciences (*supra*). The agents may be formulated as solution aerosols, dispersion or suspension aerosols of dry powders, emulsions or semisolid preparations. The aerosol may be delivered using any propellant system known to those skilled in the art. The aerosols may be applied to the upper respiratory tract, for example by nasal inhalation, or to the lower respiratory tract or to both. The glycosaminoglycan, salt or DNase may delivered using liposomes and nano-particle delivery methods. Liposomes, particularly cationic liposomes, may be used in carrier formulations.

Medicaments for use in accordance with the present invention, may include, in addition to active ingredient, a pharmaceutically acceptable excipient, carrier, buffer, stabiliser or other materials well known to those skilled in the art. In particular they may include a pharmaceutically acceptable excipient. Such materials should be nontoxic and should not interfere with the efficacy of the active ingredient. The precise nature of the carrier or other material will depend on the route of administration. Suitable pharmaceutical carriers are described in Remington's Pharmaceutical Sciences (*supra*).

The medicaments and compositions of the invention may, for example, be buffered in such a way, if they include a DNase I, that they will have a pH similar or identical to the pH optimum of the particular DNase I being employed. For example, the pH employed may be within 2, preferably 1, more preferably 0.5, even more preferably 0.2 and still more preferably 0.1 pH units of the pH optimum of the enzyme being employed. In some cases, a DNAse I may be active over a wide range of pH and the pH chosen will be that closest to physiological pH, or at least the enzyme will be active at least partially at physiological pH and hence may be buffered at physiological pH or at least a pH within 2, preferably 1, more preferably 0.5, even more preferably 0.2 and still more preferably 0.1 pH units of optimal pH.

The medicaments may include various constituents to optimise their suitability for the particular delivery route chosen. The viscosity of the medicaments may be maintained at a desired level using a pharmaceutically acceptable thickening agent. Thickening agents that can be used include methyl cellulose, xanthan gum, carboxymethyl cellulose, hydroxypropyl cellulose, carbomer, polyvinyl alcohol, alginates, acacia, chitosans and combinations thereof. The concentration of the thickening agent will depend upon the agent selected and the viscosity desired.

In some embodiments, and in particular where intranasal delivery is to be used, the medicaments may comprise a humectant. This may help reduce or prevent drying of the mucus membrane and to prevent irritation of the membranes. Suitable humectants include sorbitol, mineral oil, vegetable oil and glycerol; soothing agents; membrane conditioners; sweeteners; and combinations thereof.

The medicaments may comprise a surfactant. Suitable surfactants include nonionic, anionic and cationic surfactants. Examples of surfactants that may be used include, for example, polyoxyethylene derivatives of fatty acid partial esters of sorbitol anhydrides, such as for example, Tween 80, Polyoxyl 40 Stearate, Polyoxy ethylene 50 Stearate, fusieates, bile salts and Octoxynol.

The medicaments of the present invention may be delivered by any device adapted to introduce one or more therapeutic compositions into the upper and/or lower respiratory tract. The devices herein described may be metered-dose inhalers. The devices may be adapted to deliver the therapeutic compositions of the invention in the form of a finely dispersed mist of liquid, foam or powder. The device may use a piezoelectric effect or ultrasonic vibration to dislodge powder attached on a surface such as a tape in order to generate mist suitable for inhalation. The devices may use any

propellant system known to those in the art including, but not limited to, pumps, liquefied-gas, compressed gas and the like.

Devices herein described typically comprise a container with one or more valves through which the flow of the therapeutic composition travels and an actuator for controlling the flow. Suitable devices herein described may be seen, for example, in Remington's Pharmaceutical Sciences (*supra*). The devices suitable for administering the medicaments of the invention include inhalers and nebulisers such as those typically used to deliver steroids to asthmatics. In some cases, where the subject is for example a child, a spacer may be used to facilitate effective administration from the inhaler.

Various designs of inhalers are available commercially and may be employed to deliver the medicaments of the invention. These include the Accuhaler, Aerohaler, Aerolizer, Airmax, Autohaler, Clickhaler, Diskhaler, Easi-breathe inhaler, Fisonair, Integra, Jet inhaler, Miat-haler, Novolizer inhaler, Pulvinal inhaler, Rotahaler, Spacehaler, Spinhaler, Syncroner inhaler and Turbohaler devices.

In cases where the glycosaminoglycan and/or DNAse I are administered in the form of particles or droplets, the particle/droplet size and/or other properties of the particle/droplet may be chosen to ensure that the particles are delivered to a particular region of the respiratory tract. For example, they may be designed to reach only the upper or lower parts of the respiratory tract. In cases where the glycosaminoglycan, salt or therapeutic agent are delivered in an aqueous form preferably the solution will be isotonic to help ensure effective delivery to the subject.

It is desired to administer the medicaments to, or via, the respiratory tract the particle size of the medicament may be chosen on basis of the desired part of the respiratory tract which it is desired to administer the medicament to. In particular particles with a diameter of 10 μM are thought to be effective in reaching the lower parts of the respiratory tract and hence may be employed where such a site is the desired target for the medicaments. It is desired to deliver the medicament to the lower parts of the respiratory tract, such as alveoli for example, the diameter of the particles administered may be less than 10 μM, preferably less than 8 μM, more preferably less than 6 μM and even more preferably less than 4 μM. In a preferred embodiment the particles may have a diameter of 3 μM or less and more preferably may have a diameter of 2μM or less. The particles may have a diameter of from 3 to 5μM. In some cases the particles administered may be less than 1000 nm, preferably less than 500 nm, more preferably less than 250 nm and still more preferably less than 100 nm in diameter. The sizes may refer to particles of solid matter or droplets of solutions and suspensions.

The size of particles necessary to penetrate to a specific part of the respiratory tract will be known in the art and hence the particle size can be chosen to suit the target size. Techniques such as milling may be used to produce the very small particles necessary. In some cases the desired part of the respiratory tract may be the upper respiratory tract and hence larger particles sizes may be employed. The density of the particles and their shape may also be chosen to facilitate their delivery to the desired site.

The medicaments herein described may take a variety of forms. In case where they are to be administered via the respiratory tract they may be in the form of powders, powder microspheres, solutions, suspensions, gels, nanoparticle suspensions, liposomes, emulsions or microemulsions. The liquids present may be water or other suitable solvents such as a CFC or HFA. In the case of solutions and suspensions these may be aqueous or involve solutions other than water.

The compositions of the invention may also be coated onto implants, devices or materials to be introduced into the body. In such embodiments they may be released from the surface of the object, preferably slowly.

The present invention also provides a pharmaceutical composition comprising:

a glycosaminoglycan or a physiologically acceptable salt thereof which has an average molecular weight of from 8 to 40 kd;

- a DNAse I; and
- a pharmaceutically acceptable excipient.

The excipient may be any of those mentioned herein, or any of the carriers. The DNase I and glycosaminoglycan or salt are those as defined in claims 1-21.

*Non-clinical applications*

Although the principle use of the invention is in a clinical context, DNase I is also used for a number of non-clinical applications. Thus the demonstration of the synergistic effect of glycosaminoglycan on DNase I activity means that the amount of DNase I necessary in such applications may be decreased or alternatively that a higher level of DNase I activity may be achievable using the same amount of DNase I.

Thus the present invention provides for the use of a glycosaminoglycan, or a physiologically acceptable salt thereof which has an average molecular weight of from 8 to 40kd to increase the activity of a DNase I *in vitro.*

The non-clinical uses of the invention include, for example, in laboratory experiments and/or research. For,

example DNase I is sometimes used to remove contaminating DNA from RNA preparations and in particular from mRNA prior to cDNA synthesis. It is also used to reduce the viscosity in microinjection chambers for embryo micro-injection and other systems where increases in viscosity due to the presence of DNA may cause problems. The invention may be used to increase DNase I activity in situations where the enzyme is being used to reduce viscosity of a solution by cleaving DNA.

The products and methods of the invention may be used in any non-clinical application involving DNase I. Additional purification steps may be introduced into procedures to remove the glycosaminoglycan, if so desired or necessary, subsequent to or following the DNase I treatment. Any of the parameters described herein in relation to the clinical applications of the invention may be equally applicable to the non-clinical applications and *vice versa.*

*Kits*

Also referred to are kits comprising: a glycosaminoglycan or a salt thereof, of average molecular weight of from 8 to 40kd; a DNase I; and packaging. The glycosaminoglycan or salt and the DNase I may be provided in the kits in any of the forms discussed herein. The kit may also comprise additional reagents or assays for performing the various assays and techniques which may be performed using DNase I and in particular laboratory techniques using the enzyme. The kit may also additionally comprise instructions on how to perform the techniques.

Herein described is a kit comprising:

- a glycosaminoglycan or a physiologically acceptable salt thereof, the glycosaminoglycan or salt thereof having an average molecular weight of from 8 to 40kd;
- a DNase I; and
- packaging.

The kit may comprise instructions for the sequential, simultaneous or separate administration of the DNase I and glycosaminoglycan to a subject suffering from a disorder characterised by the presence of endogenous extracellular DNA and being treated with a glycosaminoglycan or a physiologically acceptable salt thereof, having an average molecular weight of from 8 to 40kd, so the glycosaminoglycan or salt thereof increases the activity of the DNase I where the disorder is: (a) a respiratory disorder characterised by the presence of endogenous extracellular DNA in the lung; (b) selected from cystic fibrosis, chronic airflow limitation and pneumonia; (c) Systemic lupus erythematosus (SLE); or (d) a disorder characterised by the presence of endogenous extracellular DNA at an inflammatory site.

The instructions may take the form of a label on the packing. The kit may additionally comprise means for administering the glycosaminoglycan or salt thereof and/or the DNase I such as any of those discussed herein.

The following Examples illustrate the invention.

**<u>Example 1: Demonstration of synergy of DNase and heparin *in vitro*</u>**

The effect of heparin on the activity of DNase *in vitro* was determined. Calf thymus DNA (5 μg/ml) was incubated with a range of DNase concentrations in the presence or absence of unfractionated heparin (160 USP units per mg of a heparin sodium salt from porcine intestinal mucosa - obtained from Calbiochem, catalogue number 375095) was used at a concentration of 1 mg/ml for 1 hour at 37°C. All solutions were prepared in sodium phosphate buffer containing 1.2mM magnesium sulphate. Following DNase treatment the amount of intact DNA was estimated using Hoechst stain and quantifying the associated fluorescence (Labarca & Paider, 1980, Anal. Biochem., 102:344-352). Bovine DNase I was used and had 2,500 kunitz units per mg (the same DNase was used in Examples 2 and 3). The results obtained are shown in Figure 1.

The presence of heparin significantly increased the activity of the DNase, resulting in a more rapid breakdown of the DNA. However, experiments using low molecular weight heparin of average molecular weight 6kd in the concentration range 0.01 μg/ml to 10 mg/ml had no effect on DNase activity (LMWH - a sodium heparin salt from porcine intestinal mucosa with MW6000 was employed - obtained from Sigma, catalogue number D7037). Thus, the results show that at concentrations of DNase typically achieved in the airways of cystic fibrosis patients receiving inhaled

## Claims

1. Use of a glycosaminoglycan or a physiologically acceptable salt thereof in the manufacture of a medicament for treating a subject with a disorder **characterised by** the presence of endogenous extracellular DNA, wherein the subject is also being treated with a DNase I, the glycosaminoglycan or salt thereof has an average molecular weight of from 8 to 40 kd and the glycosaminoglycan or salt thereof increases the activity of the DNase I, where the disorder is:

   (a) a respiratory disorder **characterised by** the presence of endogenous extracellular DNA in the lung;
   (b) selected from cystic fibrosis, chronic airflow limitation and pneumonia;
   (c) Systemic lupus erythematosus (SLE);or
   (d) a disorder **characterised by** the presence of endogenous extracellular DNA at an inflammatory site.

2. Use of a DNase I in the manufacture of a medicament for treating a subject with a disorder **characterised by** the presence of endogenous extracellular DNA, wherein the subject is also being treated with a glycosaminoglycan or a physiologically acceptable salt thereof, the glycosaminoglycan or salt thereof has an average molecular weight

of from 8 to 40kd and the glycosaminoglycan or salt thereof increases the activity of the DNase I, where the disorder is:

(a) a respiratory disorder **characterised by** the presence of endogenous extracellular DNA in the lung;
(b) selected from cystic fibrosis, chronic airflow limitation and pneumonia;
(c) Systemic lupus erythematosus (SLE);or
(d) a disorder **characterised by** the presence of endogenous extracellular DNA at an inflammatory site.

**3.** Use according to claim 1 or 2, wherein the glycosaminoglycan or the physiologically acceptable salt comprises repeating disaccharide units of general formula (1)

-[A-B]- (1)

wherein:

each A is the same or different and represents a moiety of formula (i) or (ii)

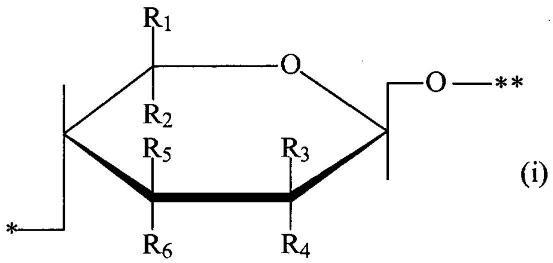
(i)

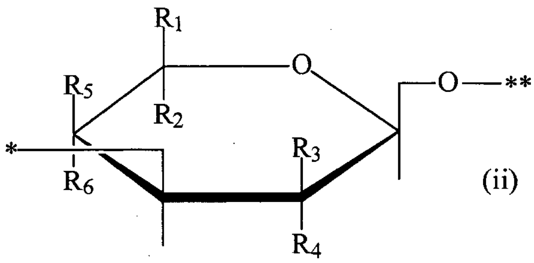
(ii)

wherein:

- one of $R_1$ and $R_2$ is hydrogen, and the other is $-C_2H$, $-SO_3H$ or $-CH_2OR$
- wherein R is hydrogen or $-SO_3H$;
- one of $R_3$ and $R_4$ is hydrogen, and the other is -OR wherein R is hydrogen or
- $-SO_3H$;
- one of $R_5$ and $R_6$ is hydrogen, and the other is -OH;
- * represents a direct bond to an adjacent hydrogen atom or B moiety; and
- ** represents a direct bond to an adjacent B moiety;

each B is the same or different and represents a moiety of formula (iii) or (iv)

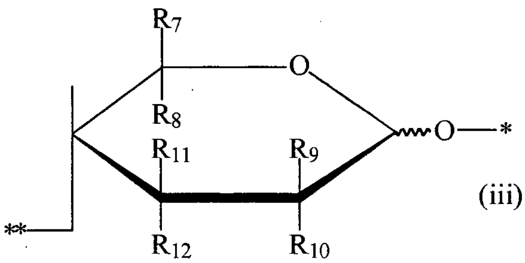
(iii)

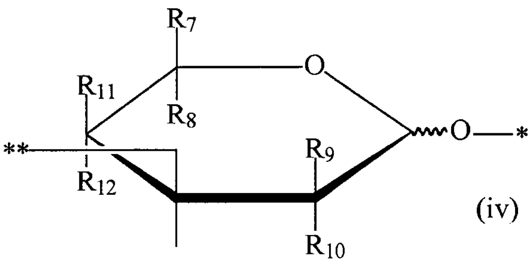
(iv)

wherein:

- one of $R_7$ and $R_8$ is hydrogen and the other is $-CH_2OH$ or $-CH_2OSO_3H$;
- one of $R_9$ and $R_{10}$ is hydrogen and the other is -NHAc, $-NH_2$ or $-NHSO_3H$;
- one of $R_{11}$ and $R_{12}$ is hydrogen and the other is -OH or $-OSO_3H$;

 indicates a bond in either stereochemical orientation;

- * represents a direct bond to a hydrogen atom or an adjacent A moiety;
- ** represents a direct bond to an adjacent A moiety.

or a physiologically acceptable salt thereof.

**4.** Use according to any one of the preceding claims, wherein the glycosaminoglycan is:

(a) a heparin; or
(b) chondroitin sulphate A, chondroitin sulphate C, chondroitin sulphate D, chondroitin sulphate E, hyaluronic acid, keratan sulphate, or heparan sulphate.

**5.** Use according to any one of the preceding claims, wherein the sodium salt of heparin or heparin sulphate is used.

**6.** Use according to any one of the preceding claims wherein the DNase I:

(a) has the amino acid sequence of SEQ ID NO:2 or is a DNase I with more than 65% amino acid sequence identity to the amino acid sequence of SEQ ID NO:2 and which retains hydrolytic activity;
(b) is a human type I DNase; and/or
(c) is a recombinant DNase I.

**7.** Use according to any one of the preceding claims, wherein the glycosaminoglycan or salt has:

(a) an average molecular weight of from 12 to 18 kd;
(b) anti-coagulant activity; and/or
(c) not been subjected to fragmentation and/or depolymerisation.

**8.** Use according to any one of the preceding claims, wherein the ratio of the amount of glycosaminoglycan or salt thereof to the amount of DNase I administered is from 5000:1 to 1:50 unit for unit.

**9.** Use according to claim 8, wherein the ratio is from 25:1 to 1:25.

**10.** Use according to any one of the previous claims, wherein:

(a) the glycosaminoglycan or salt thereof and the DNase I are administered at the same time;
(b) the glycosaminoglycan or salt thereof is administered before or after the DNase I; and/or
(c) the glycosaminoglycan or salt thereof and the DNase I are administered as separate medicaments.

**11.** Use according to any one of the preceding claims, wherein the subject has an $FEV_1$ of less than 75% of the expected value for an equivalent subject not having the disorder.

**12.** Use according any one of the preceding claims, wherein the glycosaminoglycan, or salt and/or the DNase I are administered via inhalation, intra-nasally and/or via instillation.

**13.** A pharmaceutical composition comprising:

- a glycosaminoglycan or a physiologically acceptable salt thereof which has an average molecular weight of from 8 to 40 kd;
- a DNase I; and
- a pharmaceutically acceptable excipient, carrier or dilutent.

**14.** A pharmaceutical composition according to claim 13 which is in the form of a dry powder.

**15.** Products comprising a glycosaminoglycan or a physiologically acceptable salt thereof, the glycosaminoglycan or salt thereof having an average molecular weight of from 8 to 40kd, and a DNase I for use in the treatment of a disorder **characterised by** the presence of endogenous extracellular DNA in the subject to be treated, where the glycosaminoglycan or salt thereof increases the activity of the DNase I and wherein the products are to be administered in a simultaneous, separate or sequential manner, where the disorder is:

(a) a respiratory disorder **characterised by** the presence of endogenous extracellular DNA in the lung;
(b) selected from cystic fibrosis, chronic airflow limitation and pneumonia;
(c) Systemic lupus erythematosus (SLE);or
(d) a disorder **characterised by** the presence of endogenous extracellular DNA at an inflammatory site.

**16.** Products for use according to claim 15, wherein:

(A) the glycosaminoglycan or the physiologically acceptable salt thereof comprises repeating disaccharide units of general formula (1)

-[A-B]- (1)

wherein :

each A is the same or different and represents a moiety of formula (i) or (ii)

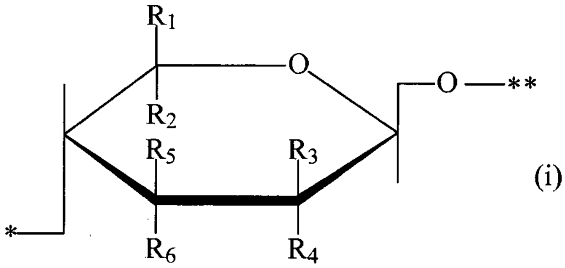
(i)

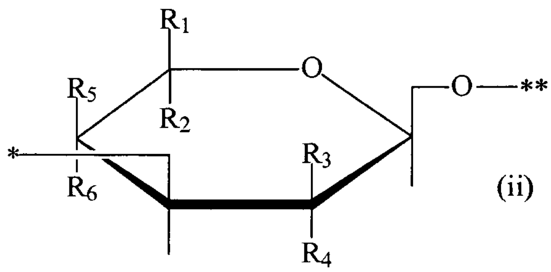
(ii)

wherein:

- one of $R_1$ and $R_2$ is hydrogen, and the other is $-C_2H$, $-SO_3H$ or $-CH_2OR$
- wherein R is hydrogen or $-SO_3H$;
- one of $R_3$ and $R_4$ is hydrogen, and the other is -OR wherein R is hydrogen or
- $-SO_3H$;
- one of $R_5$ and $R_6$ is hydrogen, and the other is -OH;
- * represents a direct bond to an adjacent hydrogen atom or B moiety; and
- ** represents a direct bond to an adjacent B moiety;

each B is the same or different and represents a moiety of formula (iii) or (iv)

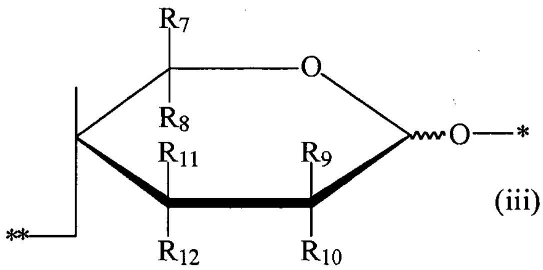
(iii)

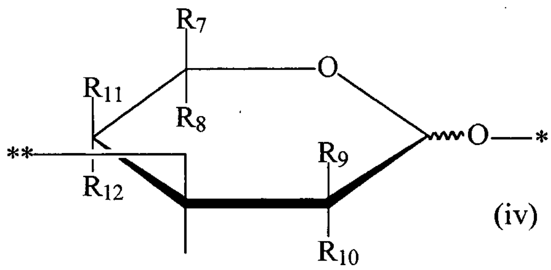
(iv)

wherein:

- one of $R_7$ and $R_8$ is hydrogen and the other is $-CH_2OH$ or $-CH_2OSO_3H$;
- one of $R_9$ and $R_{10}$ is hydrogen and the other is -NHAc, $-NH_2$ or $-NHSO_3H$;
- one of $R_{11}$ and $R_{12}$ is hydrogen and the other is -OH or $-OSO_3H$; indicates a bond in either stereochemical orientation;

- * represents a direct bond to a hydrogen atom or an adjacent A moiety;
- ** represents a direct bond to an adjacent A moiety.

or a physiologically acceptable salt thereof; or
(B) the glycosaminoglycan or salt thereof is a heparin; or
(C) the glycosaminoglycan or salt thereof is chondroitin sulphate A, chondroitin sulphate C, chondroitin sulphate D, chondroitin sulphate E hyaluronic acid, keratan sulphate, or heparan sulphate; or
(D) the sodium salt of heparin or heparin sulphate is used; or
(E) the glycosaminoglycan or salt thereof has an average molecular weight of from 12 to 18 kd; or
(F) the glycosaminoglycan or salt thereof has anti-coagulant activity; or
(G) the glycosaminoglycan or salt thereof has not been subjected to fragmentation and/or depolymerisation; or
(H) the DNase I has the amino acid sequence of SEQ ID NO:2 or is a DNase I with more than 65% amino acid sequence identity to the amino acid sequence of SEQ ID NO:2;
(I) the DNase I is a human type I DNase; or
(J) the DNase I is a recombinant DNase I; or
(K) the ratio of the amount of glycosaminoglycan or salt thereof to the amount of DNase I administered is from 5000:1 to 1:50 unit for unit; or
(L) the ratio of the amount of glycosaminoglycan or salt thereof to the amount of DNase I to be administered is from 25:1 to 1:25.

**17.** An agent for use in treating a subject having a disorder **characterised by** the presence of endogenous extracellular DNA, the agent comprising a glycosaminoglycan or a physiologically acceptable salt thereof, the glycosaminoglycan or salt thereof having an average molecular weight of from 8 to 40kd, wherein the said subject is being treated with a DNase I and the glycosaminoglycan or salt thereof increases the activity of the DNase I, where the disorder is:

(a) a respiratory disorder **characterised by** the presence of endogenous extracellular DNA in the lung;
(b) selected from cystic fibrosis, chronic airflow limitation and pneumonia;
(c) Systemic lupus erythematosus (SLE);or
(d) a disorder **characterised by** the presence of endogenous extracellular DNA at an inflammatory site.

**18.** An agent for use in treating a subject having a disorder **characterised by** the presence of endogenous extracellular DNA, the agent comprising a DNase I, wherein the said subject is being treated with a glycosaminoglycan or a physiologically acceptable salt thereof, the glycosaminoglycan or salt thereof having an average molecular weight of from 8 to 40kd and the glycosaminoglycan or salt thereof increases the activity of the DNase I, where the disorder is:

(a) a respiratory disorder **characterised by** the presence of endogenous extracellular DNA in the lung;
(b) selected from cystic fibrosis, chronic airflow limitation and pneumonia;
(c) Systemic lupus erythematosus (SLE);or
(d) a disorder **characterised by** the presence of endogenous extracellular DNA at an inflammatory site.

**19.** A pharmaceutical composition according to claim 13 or 14 or an agent for use according to claim 17 or 18, wherein:

(A) the glycosaminoglycan or the physiologically acceptable salt thereof comprises repeating disaccharide units of general formula (1)

-[A-B]- (1)

wherein :

each A is the same or different and represents a moiety of formula (i) or (ii)

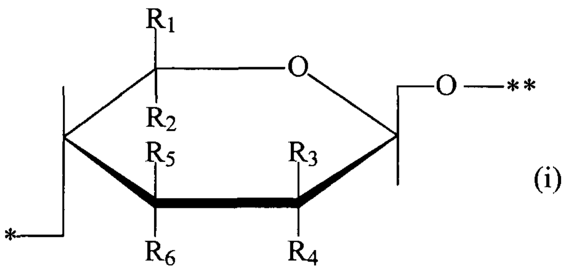

(i)

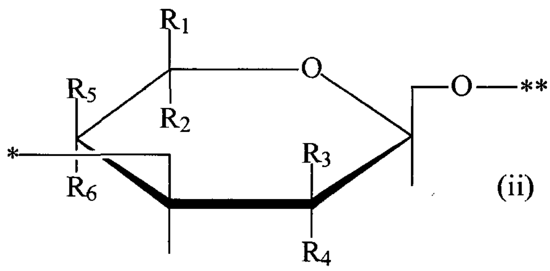

(ii)

wherein:

- one of $R_1$ and $R_2$ is hydrogen, and the other is $-C_2H$, $-SO_3H$ or $-CH_2OR$
- wherein R is hydrogen or $-SO_3H$;
- one of $R_3$ and $R_4$ is hydrogen, and the other is -OR wherein R is hydrogen or
- $SO_3H$;
- one of $R_5$ and $R_6$ is hydrogen, and the other is -OH;
- * represents a direct bond to an adjacent hydrogen atom or B moiety; and
- ** represents a direct bond to an adjacent B moiety;

each B is the same or different and represents a moiety of formula (iii) or (iv)

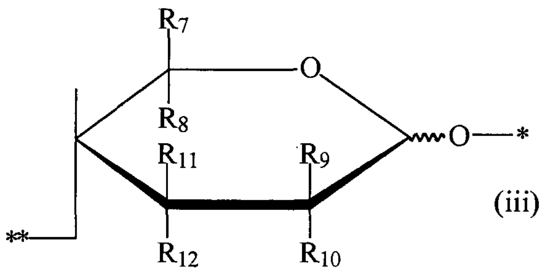

(iii)

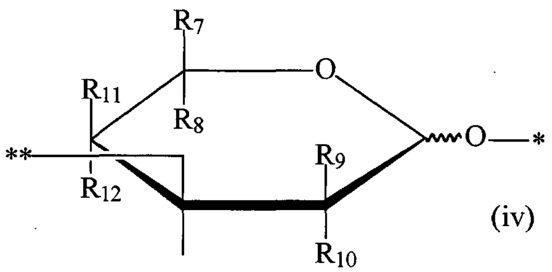

(iv)

wherein:

- one of $R_7$ and $R_8$ is hydrogen and the other is $-CH_2OH$ or $-CH_2OSO_3H$;
- one of $R_9$ and $R_{10}$ is hydrogen and the other is -NHAc, $-NH_2$ or $-NHSO_3H$;
- one of $R_{11}$ and $R_{12}$ is hydrogen and the other is -OH or $-OSO_3H$; ∿∿ indicates a bond in either stereochemical orientation;

- * represents a direct bond to a hydrogen atom or an adjacent A moiety;
- ** represents a direct bond to an adjacent A moiety.

or a physiologically acceptable salt thereof; or
(B) the glycosaminoglycan or salt thereof is a heparin; or
(C) the glycosaminoglycan or salt thereof is chondroitin sulphate A, chondroitin sulphate C, chondroitin sulphate D, chondroitin sulphate E hyaluronic acid, keratan sulphate, or heparan sulphate; or
(D) the sodium salt of heparin or heparin sulphate is used; or
(E) the glycosaminoglycan or salt thereof has an average molecular weight of from 12 to 18 kd; or
(F) the glycosaminoglycan or salt thereof has anti-coagulant activity; or
(G) the glycosaminoglycan or salt thereof has not been subjected to fragmentation and/or depolymerisation; or
(H) the DNase I has the amino acid sequence of SEQ ID NO:2 or is a DNase I with more than 65% amino acid sequence identity to the amino acid sequence of SEQ ID NO:2;
(I) the DNase I is a human type I DNase; or
(J) the DNase I is a recombinant DNase I; or

(K) the ratio of the amount of glycosaminoglycan or salt thereof to the amount of DNase I administered is from 5000:1 to 1:50 unit for unit; or

(L) the ratio of the amount of glycosaminoglycan or salt thereof to the amount of DNase I to be administered is from 25:1 to 1:25.

**20.** A nebuliser or other liquid aerosol device, dry powder inhaler or metered dose inhaler comprising a composition comprising:

- a glycosaminoglycan or a physiologically acceptable salt thereof which has an average molecular weight of from 8 to 40kd;
- a DNAse I; and
- a pharmaceutically acceptable excipient, carrier or dilutent.

**21.** Use of a glycosaminoglycan or a physiologically acceptable salt thereof, the glycosaminoglycan or salt thereof having an average molecular weight of from 8 to 40kd to increase the activity of a DNase I *in vitro*.

**Patentansprüche**

**1.** Verwendung eines Glycosaminoglycans oder eines physiologisch annehmbaren Salzes davon in der Herstellung eines Medikaments zur Behandlung eines Subjekts mit einer Störung, die durch Vorliegen von endogener extrazellulärer DNA gekennzeichnet ist, wobei das Subjekt auch mit einer DNase I behandelt wird, das Glycosaminoglycan oder Salz davon ein durchschnittliches Molekulargewicht von 8 bis 40 kD hat und das Glycosaminoglycan oder Salz davon die Aktivität der DNase I erhöht, wobei die Störung ist:

(a) eine respiratorische Störung, die durch das Vorliegen von endogener extrazellulärer DNA in der Lunge gekennzeichnet ist;

(b) ausgewählt aus zystischer Fibrose, chronischer Luftstrombeschränkung und Pneumonie;

(c) systemischer Lupus erythematodes (SLE) oder

(d) eine Störung, die durch das Vorliegen von endogener extrazellulärer DNA an einer Entzündungsstelle gekennzeichnet ist.

**2.** Verwendung einer DNase I in der Herstellung eines Medikaments zur Behandlung eines Subjekts mit einer Störung, die durch das Vorliegen von endogener extrazellulärer DNA gekennzeichnet ist, wobei das Subjekt auch mit einem Glycosaminoglycan oder einem physiologisch annehmbaren Salz davon behandelt wird, das Glycosaminoglycan oder Salz davon ein durchschnittliches Molekulargewicht von 8 bis 40 kD hat und das Glycosaminoglycan oder Salz davon die Aktivität der DNase I erhöht, wobei die Störung ist:

(a) eine respiratorische Störung, die durch das Vorliegen von endogener extrazellulärer DNA in der Lunge gekennzeichnet ist;

(b) ausgewählt aus zystischer Fibrose, chronischer Luftstrombeschränkung und Pneumonie;

(c) systemischer Lupus erythematodes (SLE) oder

(d) eine Störung, die durch das Vorliegen von endogener extrazellulärer DNA an einer Entzündungsstelle gekennzeichnet ist.

**3.** Verwendung nach Anspruch 1 oder 2, wobei das Glycosaminoglycan oder das physiologisch annehmbare Salz Disaccharid-Repetiereinheiten der allgemeinen Formel (1)

$$-[A\text{-}B]- \qquad (1)$$

umfasst, wobei:

A jeweils gleich oder verschieden ist und eine Gruppierung der Formel (i) oder (ii) darstellt

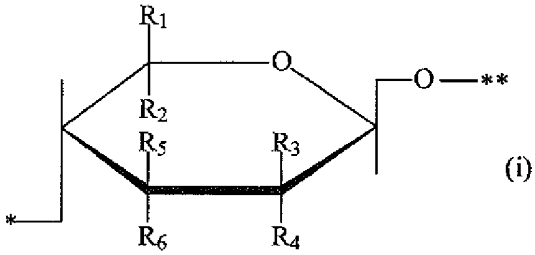

(i)

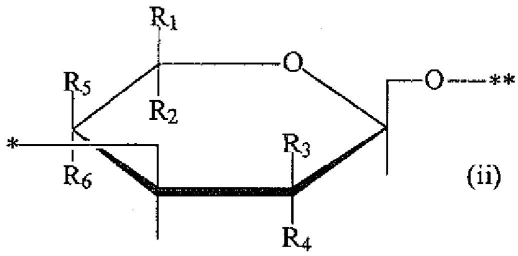

(ii)

wobei:

- eins von $R_1$ und $R_2$ Wasserstoff ist und das andere $-C_2H$, $-SO_3H$ oder $-CH_2OR$ ist,
- worin R Wasserstoff oder $-SO_3H$ ist;
- eins von $R_3$ und $R_4$ Wasserstoff ist und das andere -OR ist, worin R Wasserstoff oder $-SO_3H$ ist;
- eins von $R_5$ und $R_6$ Wasserstoff ist und das andere -OH ist;
- * eine direkte Bindung an ein benachbartes Wasserstoffatom oder eine benachbarte B-Gruppierung darstellt und
- ** eine direkte Bindung an eine benachbarte B-Gruppierung darstellt;

B jeweils gleich oder verschieden ist und eine Gruppierung der Formel (iii) oder (iv) darstellt

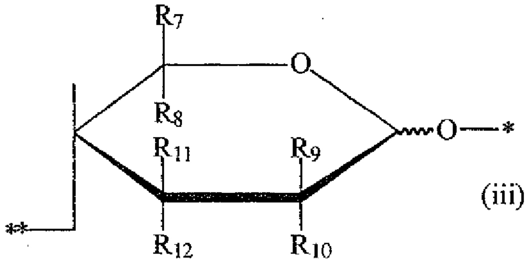

(iii)

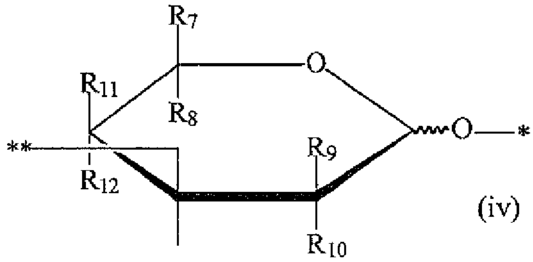

(iv)

wobei:

- eins von $R_7$ und $R_8$ Wasserstoff ist und das andere $-CH_2OH$ oder $-CH_2OSO_3H$ ist;
- eins von $R_9$ und $R_{10}$ Wasserstoff ist und das andere -NHAc, $-NH_2$ oder $-NHSO_3H$ ist;
- eins von $R_{11}$ und $R_{12}$ Wasserstoff ist und das andere -OH oder $-OSO_3H$ ist;

∿∿∿ eine Bindung in jeder stereochemischen Orientierung anzeigt;

- * eine direkte Bindung an ein Wasserstoffatom oder eine benachbarte A-Gruppierung darstellt;
- ** eine direkte Bindung an eine benachbarte A-Gruppierung darstellt;

oder ein physiologisch annehmbares Salz davon.

**4.** Verwendung nach einem der vorangehenden Ansprüche, wobei das Glycosaminoglycan ist:

(a) ein Heparin oder
(b) Chondroitinsulfat A, Chondroitinsulfat C, Chondroitinsulfat D, Chondroitinsulfat E, Hyaluronsäure, Keratansulfat oder Heparansulfat.

**5.** Verwendung nach einem der vorangehenden Ansprüche, wobei das Natriumsalz von Heparin oder Heparinsulfat verwendet wird.

**6.** Verwendung nach einem der vorangehenden Ansprüche, wobei die DNase I:

(a) die Aminosäuresequenz von SEQ ID NO: 2 hat oder eine DNase I mit mehr als 65% Aminosäuresequenzidentität zu der Aminosäuresequenz von SEQ ID NO: 2 ist und die hydrolytische Aktivität beibehält;
(b) eine humaner Typ I DNase ist und/oder
(c) eine rekombinante DNase I ist.

**7.** Verwendung nach einem der vorangehenden Ansprüche, wobei das Glycosaminoglycan oder Salz hat:

(a) ein durchschnittliches Molekulargewicht von 12 bis 18 kD;
(b) Antikoagulans-Aktivität und/oder
(c) keiner Fragmentierung und/oder Depolymerisation unterzogen wurde.

**8.** Verwendung nach einem der vorangehenden Ansprüche, wobei das Verhältnis der Menge an Glycosaminoglycan oder Salz davon zu der Menge an DNase I, die verabreicht werden, 5000:1 bis 1:50 Einheit für Einheit ist.

**9.** Verwendung nach Anspruch 8, wobei das Verhältnis 25:1 bis 1:25 ist.

**10.** Verwendung nach einem der vorangehenden Ansprüche, wobei:

(a) das Glycosaminoglycan oder Salz davon und die DNase I gleichzeitig verabreicht werden;
(b) das Glycosaminoglycan oder Salz davon vor oder nach der DNase I verabreicht wird und/oder
(c) das Glycosaminoglycan oder Salz davon und die DNase I als getrennte Medikamente verabreicht werden.

**11.** Verwendung nach einem der vorangehenden Ansprüche, wobei das Subjekt einen $FEV_1$ von weniger als 75% des erwarteten Wertes für ein äquivalentes Subjekt, das die Störung nicht hat, hat.

**12.** Verwendung nach einem der vorangehenden Ansprüche, wobei das Glycosaminoglycan oder Salz und/oder die DNase I durch Inhalation, intranasal und/oder durch Instillation verabreicht werden.

**13.** Pharmazeutische Zusammensetzung, umfassend:

- ein Glycosaminoglycan oder ein physiologisch annehmbares Salz davon, das ein durchschnittliches Molekulargewicht von 8 bis 40 kD hat;
- eine DNase I und
- ein pharmazeutisch annehmbares Exzipiens, einen pharmazeutisch annehmbaren Träger oder ein pharmazeutisch annehmbares Verdünnungsmittel.

**14.** Pharmazeutische Zusammensetzung nach Anspruch 13, welche in der Form eines trockenen Pulvers ist.

**15.** Produkte, umfassend ein Glycosaminoglycan oder ein physiologisch annehmbares Salz davon, wobei das Glycosaminoglycan oder Salz davon ein durchschnittliches Molekulargewicht von 4 bis 80 kD hat, und eine DNase I zur Verwendung bei der Behandlung einer Störung, die durch das Vorliegen von endogener extrazellulärer DNA bei dem zu behandelnden Subjekt gekennzeichnet ist, wobei das Glycosaminoglycan oder Salz davon die Aktivität der DNase I erhöht und wobei die Produkte in gleichzeitiger, getrennter oder sequenzieller Art zu verabreichen sind, wobei die Störung ist:

(a) eine respiratorische Störung, die durch das Vorliegen von endogener extrazellulärer DNA in der Lunge gekennzeichnet ist;
(b) ausgewählt aus zystischer Fibrose, chronischer Luftstrombeschränkung und Pneumonie;
(c) systemischer Lupus erythematodes (SLE) oder
(d) eine Störung, die durch das Vorliegen von endogener extrazellulärer DNA an einer Entzündungsstelle gekennzeichnet ist.

**16.** Produkte zur Verwendung nach Anspruch 15, wobei:

(A) das Glycosaminoglycan oder das physiologisch annehmbare Salz davon Disaccharid-Repetiereinheiten der allgemeinen Formel (1)

$$-[A-B]- \qquad (1)$$

umfasst, wobei:

A jeweils gleich oder verschieden ist und eine Gruppierung der Formel (i) oder (ii) darstellt

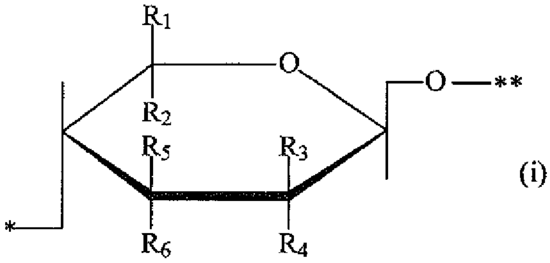
(i)

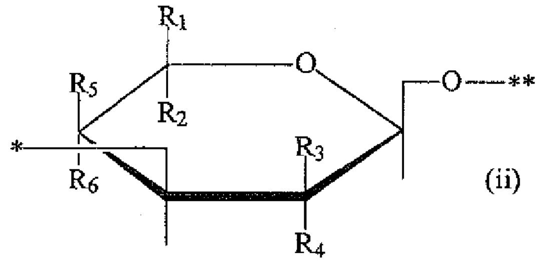
(ii)

wobei:

- eins von $R_1$ und $R_2$ Wasserstoff ist und das andere $-C_2H$, $-SO_3H$ oder $-CH_2OR$ ist,
- worin R Wasserstoff oder $-SO_3H$ ist;
- eins von $R_3$ und $R_4$ Wasserstoff ist und das andere -OR ist, worin R Wasserstoff oder $-SO_3H$ ist;
- eins von $R_5$ und $R_6$ Wasserstoff ist und das andere -OH ist;
- * eine direkte Bindung an ein benachbartes Wasserstoffatom oder eine benachbarte B-Gruppierung darstellt und
- ** eine direkte Bindung an eine benachbarte B-Gruppierung darstellt;

B jeweils gleich oder verschieden ist und eine Gruppierung der Formel (iii) oder (iv) darstellt

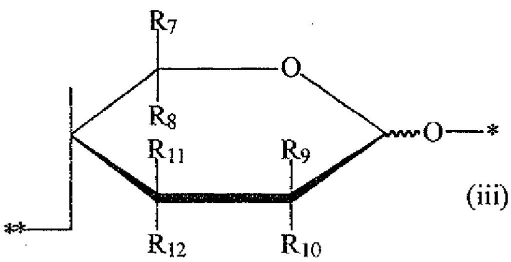
(iii)

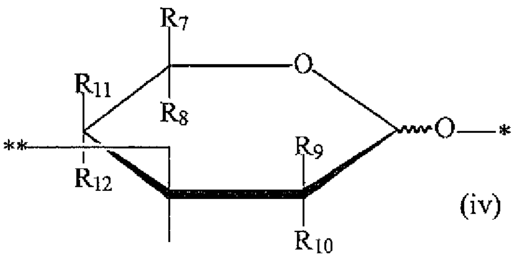
(iv)

wobei:

- eins von $R_7$ und $R_8$ Wasserstoff ist und das andere $-CH_2OH$ oder $-CH_2OSO_3H$ ist;
- eins von $R_9$ und $R_{10}$ Wasserstoff ist und das andere -NHAc, $-NH_2$ oder $-NHSO_3H$ ist;
- eins von $R_{11}$ und $R_{12}$ Wasserstoff ist und das andere -OH oder $-OSO_3H$ ist;

∿∿ eine Bindung in jeder stereochemischen Orientierung anzeigt;

- * eine direkte Bindung an ein Wasserstoffatom oder eine benachbarte A-Gruppierung darstellt;
- ** eine direkte Bindung an eine benachbarte A-Gruppierung darstellt;

oder ein physiologisch annehmbares Salz davon; oder

(B) das Glycosaminoglycan oder Salz davon ein Heparin ist oder
(C) das Glycosaminoglycan oder Salz davon Chondroitinsulfat A, Chondroitinsulfat C, Chondroitinsulfat D, Chondroitinsulfat E, Hyaluronsäure, Keratansulfat oder Heparansulfat ist oder
(D) das Natriumsalz von Heparin oder Heparinsulfat verwendet wird oder
(E) das Glycosaminoglycan oder Salz davon ein durchschnittliches Molekulargewicht von 12 bis 18 kD hat oder
(F) das Glycosaminoglycan oder Salz davon Antikoagulans-Aktivität hat oder

(G) das Glycosaminoglycan oder Salz davon keiner Fragmentierung und/oder Depolymerisation unterzogen wurde oder

(H) die DNase 1 die Aminosäuresequenz von SEQ ID NO:2 hat oder eine DNase I mit mehr als 65% Aminosäuresequenzidentität zu der Aminosäuresequenz von SEQ ID NO:2 ist;

(I) die DNase I eine humane Typ-I-DNase ist oder

(J) die DNase I eine rekombinante DNase I ist oder

(K) das Verhältnis der Menge an Glycosaminoglycan oder Salz davon zu der Menge an DNase I, die verabreicht werden, 5000:1 bis 1:50 Einheit für Einheit ist oder

(L) das Verhältnis der Menge an Glycosaminoglycan oder Salz davon zu der Menge an DNase I, die zu verabreichen sind, 25:1 bis 1:25 ist.

**17.** Mittel zur Verwendung bei der Behandlung eines Subjekts, das eine Störung hat, die durch das Vorliegen von endogener extrazellulärer DNA gekennzeichnet ist, wobei das Mittel ein Glycosaminoglycan oder ein physiologisch annehmbares Salz davon umfasst, wobei das Glycosaminoglycan oder Salz davon ein durchschnittliches Molekulargewicht von 8-40 kD hat, wobei das Subjekt mit einer DNase I behandelt wird, und das Glycosaminoglycan oder Salz davon die Aktivität der DNase I erhöht, wobei die Störung ist:

(a) eine respiratorische Störung, die durch das Vorliegen von endogener extrazellulärer DNA in der Lunge gekennzeichnet ist;

(b) ausgewählt aus zystischer Fibrose, chronischer Luftstrombeschränkung und Pneumonie;

(c) systemischer Lupus erythematodes (SLE) oder

(d) eine Störung, die durch das Vorliegen von endogener extrazellulärer DNA an einer Entzündungsstelle gekennzeichnet ist.

**18.** Mittel zur Verwendung bei der Behandlung eines Subjekts, das eine Störung hat, die durch das Vorliegen von endogener extrazellulärer DNA gekennzeichnet ist, wobei das Mittel eine DNase I umfasst, wobei das Subjekt mit einem Glycosaminoglycan oder einem physiologisch annehmbaren Salz davon behandelt wird, das Glycosaminoglycan oder Salz davon ein durchschnittliches Molekulargewicht von 8 bis 40 kD hat und das Glycosaminoglycan oder Salz davon die Aktivität der DNase I erhöht, wobei die Störung ist:

(a) eine respiratorische Störung, die durch das Vorliegen von endogener extrazellulärer DNA in der Lunge gekennzeichnet ist;

(b) ausgewählt aus zystischer Fibrose, chronischer Luftstrombeschränkung und Pneumonie;

(c) systemischer Lupus erythematodes (SLE) oder

(d) eine Störung, die durch das Vorliegen von endogener extrazellulärer DNA an einer Entzündungsstelle gekennzeichnet ist.

**19.** Pharmazeutische Zusammensetzung nach Anspruch 13 oder 14 oder Mittel zur Verwendung nach Anspruch 17 oder 18, wobei:

(A) das Glycosaminoglycan oder das physiologisch annehmbare Salz davon Disaccharid-Repetiereinheiten der allgemeinen Formel (1)

-[A-B]- (1)

umfasst, wobei:

A jeweils gleich oder verschieden ist und eine Gruppierung der Formel (i) oder (ii) darstellt

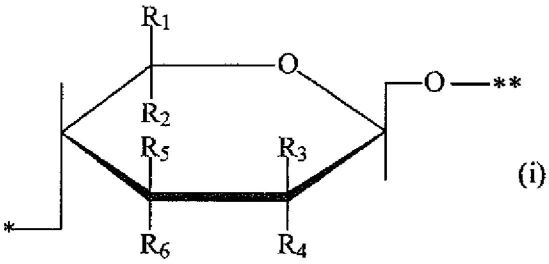
(i)
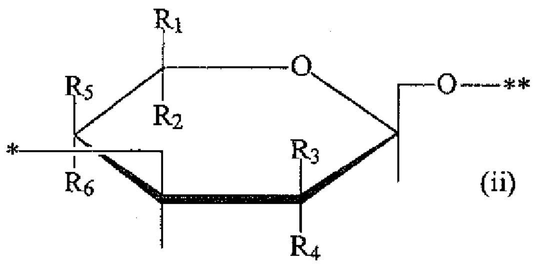
(ii)

wobei:

- eins von $R_1$ und $R_2$ Wasserstoff ist und das andere $-C_2H$, $-SO_3H$ oder $-CH_2OR$ ist,
- worin R Wasserstoff oder $-SO_3H$ ist;
- eins von $R_3$ und $R_4$ Wasserstoff ist und das andere -OR ist, worin R Wasserstoff oder $-SO_3H$ ist;
- eins von $R_5$ und $R_6$ Wasserstoff ist und das andere -OH ist;
- * eine direkte Bindung an ein benachbartes Wasserstoffatom oder eine benachbarte B-Gruppierung darstellt und
- ** eine direkte Bindung an eine benachbarte B-Gruppierung darstellt;

B jeweils gleich oder verschieden ist und eine Gruppierung der Formel (iii) oder (iv) darstellt

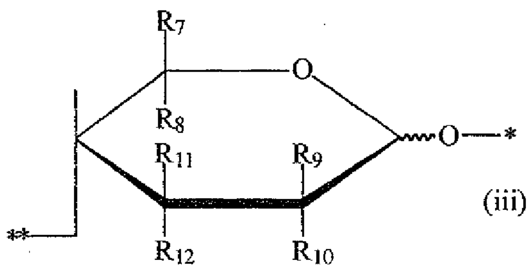
(iii)
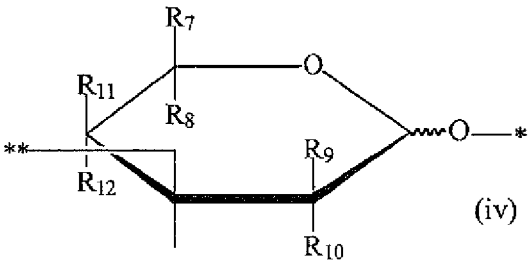
(iv)

wobei:

- eins von $R_7$ und $R_8$ Wasserstoff ist und das andere $-CH_2OH$ oder $-CH_2OSO_3H$ ist;
- eins von $R_9$ und $R_{10}$ Wasserstoff ist und das andere -NHAc, $-NH_2$ oder $-NHSO_3H$ ist;
- eins von $R_{11}$ und $R_{12}$ Wasserstoff ist und das andere -OH oder $-OSO_3H$ ist;

∿∿∿ eine Bindung in jeder stereochemischen Orientierung anzeigt;

- * eine direkte Bindung an ein Wasserstoffatom oder eine benachbarte A-Gruppierung darstellt;
- ** eine direkte Bindung an eine benachbarte A-Gruppierung darstellt;

oder ein physiologisch annehmbares Salz davon; oder
(B) das Glycosaminoglycan oder Salz davon ein Heparin ist oder
(C) das Glycosaminoglycan oder Salz davon Chondroitinsulfat A, Chondroitinsulfat C, Chondroitinsulfat D, Chondroitinsulfat E, Hyaluronsäure, Keratansulfat oder Heparansulfat ist oder
(D) das Natriumsalz von Heparin oder Heparinsulfat verwendet wird oder
(E) das Glycosaminoglycan oder Salz davon ein durchschnittliches Molekulargewicht von 12 bis 18 kD hat oder
(F) das Glycosaminoglycan oder Salz davon Antikoagulans-Aktivität hat oder
(G) das Glycosaminoglycan oder Salz davon keiner Fragmentierung und/oder Depolymerisation unterzogen wurde oder
(H) die DNase I die Aminosäuresequenz von SEQ ID NO:2 hat oder eine DNase I mit mehr als 65% Aminosäuresequenzidentität zu der Aminosäuresequenz von SEQ ID NO:2 ist;
(I) die DNase I eine humane Typ-I-DNase ist oder
(J) die DNase I eine rekombinante DNase I ist oder
(K) das Verhältnis der Menge an Glycosaminoglycan oder Salz davon zu der Menge an DNase I, die verabreicht werden, 5000:1 bis 1:50 Einheit für Einheit ist oder
(L) das Verhältnis der Menge an Glycosaminoglycan oder Salz davon zu der Menge an DNase I, die zu verabreichen sind, 25:1 bis 1:25 ist.

**20.** Vernebler oder andere Flüssigaerosolvorrichtung, Trockenpulverinhalator oder Dosierinhalator, umfassend eine Zusammensetzung, umfassend:

- ein Glycosaminoglycan oder ein physiologisch annehmbares Salz davon mit einem durchschnittlichen Molekulargewicht von 8 bis 40 kD;
- eine DNase I und
- ein pharmazeutisch annehmbares Exzipiens, einen pharmazeutisch annehmbaren Träger oder ein pharmazeutisch annehmbares Verdünnungsmittel.

**21.** Verwendung eines Glycosaminoglycan oder eines physiologisch annehmbaren Salzes davon, wobei das Glycosaminoglycan oder Salz davon ein durchschnittliches Molekulargewicht von 8 bis 40 kD hat, um die Aktivität einer DNase I *in vitro* zu erhöhen.

## Revendications

**1.** Utilisation d'un glycosaminoglycanne, ou d'un sel physiologiquement admissible d'un glycosaminoglycanne, dans la fabrication d'un médicament destiné au traitement d'un sujet atteint d'un trouble **caractérisé par** la présence d'ADN extracellulaire endogène, étant entendu que le sujet est également traité avec une ADNase I, que le glycosaminoglycanne ou son sel présente une masse molaire moyenne de 8 à 40 kDa, que le glycosaminoglycanne ou son sel renforce l'activité de l'ADNase I, et que le trouble est :

a) une maladie respiratoire **caractérisée par** la présence d'ADN extracellulaire endogène dans le poumon,
b) choisi parmi une mucoviscidose, une limitation chronique du débit respiratoire et une pneumonie,
c) un lupus érythémateux aigu disséminé,
d) ou un trouble **caractérisé par** la présence d'ADN extracellulaire endogène au niveau d'un site d'inflammation.

**2.** Utilisation d'une ADNase I dans la fabrication d'un médicament destiné au traitement d'un sujet atteint d'un trouble **caractérisé par** la présence d'ADN extracellulaire endogène, étant entendu que le sujet est également traité avec un glycosaminoglycanne ou un sel physiologiquement admissible d'un glycosaminoglycanne, que le glycosaminoglycanne ou son sel présente une masse molaire moyenne de 8 à 40 kDa, que le glycosaminoglycanne ou son sel renforce l'activité de l'ADNase I, et que le trouble est :

a) une maladie respiratoire **caractérisée par** la présence d'ADN extracellulaire endogène dans le poumon,
b) choisi parmi une mucoviscidose, une limitation chronique du débit respiratoire et une pneumonie,
c) un lupus érythémateux aigu disséminé,
d) ou un trouble **caractérisé par** la présence d'ADN extracellulaire endogène au niveau d'un site d'inflammation.

**3.** Utilisation conforme à la revendication 1 ou 2, pour laquelle le glycosaminoglycanne ou son sel physiologiquement admissible comporte des motifs répétés de disaccharide de formule générale (1) :

$$-[A-B]- \quad (1)$$

dans laquelle

- chaque symbole A représente un reste, identique ou différent, de formule (i) ou (ii) :

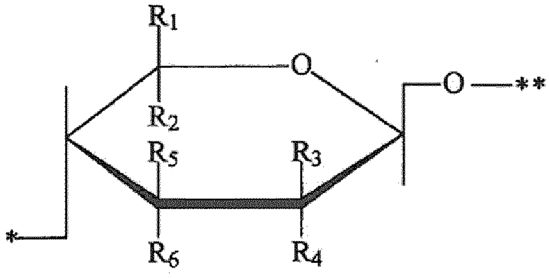

(i)

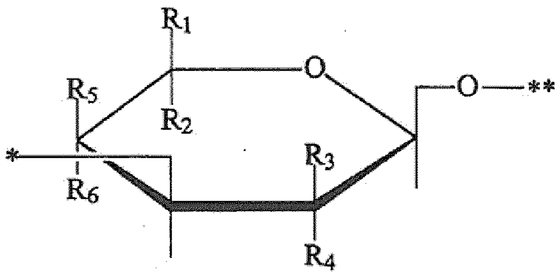

(ii)

dans lesquelles

- l'un des symboles $R_1$ et $R_2$ représente un atome d'hydrogène, et l'autre, un groupe de formule $-CO_2H$, $-SO_3H$, ou $-CH_2OR$ où R représente un atome d'hydrogène ou un groupe de formule $-SO_3H$,
- l'un des symboles $R_3$ et $R_4$ représente un atome d'hydrogène, et l'autre, un groupe de formule -OR où R représente un atome d'hydrogène ou un groupe de formule $-SO_3H$,
- l'un des symboles $R_5$ et $R_6$ représente un atome d'hydrogène, et l'autre, un groupe de formule -OH,
- l'astérisque simple * indique une liaison directe vers un atome d'hydrogène ou un reste B adjacent,
- et l'astérisque double ** indique une liaison directe vers un reste B adjacent,
- et chaque symbole B représente un reste, identique ou différent, de formule (iii) ou (iv) :

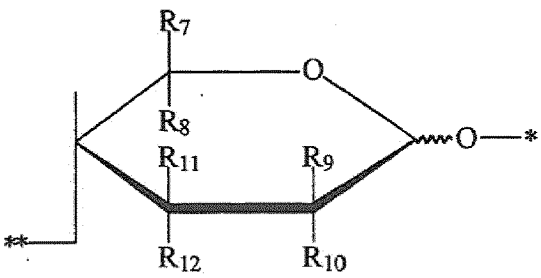

(iii)

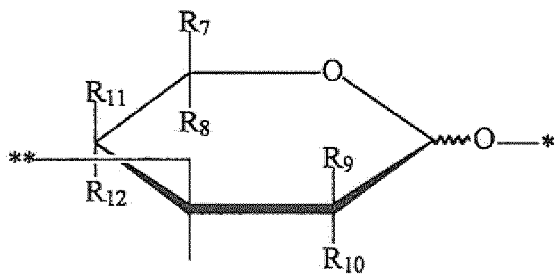

(iv)

dans lesquelles

- l'un des symboles $R_7$ et $R_8$ représente un atome d'hydrogène, et l'autre, un groupe de formule $-CH_2OH$ ou $-CH_2OSO_3H$,
- l'un des symboles $R_9$ et $R_{10}$ représente un atome d'hydrogène, et l'autre, un groupe de formule -NHAc, $-NH_2$ ou $-NHSO_3H$,
- l'un des symboles $R_{11}$ et $R_{12}$ représente un atome d'hydrogène, et l'autre, un groupe de formule -OH ou $-OSO_3H$,
- la ligne brisée ∿ indique une liaison dans l'une ou l'autre orientation stéréochimique,
- l'astérisque simple * indique une liaison directe vers un atome d'hydrogène ou un reste A adjacent,
- et l'astérisque double ** indique une liaison directe vers un reste A adj acent ;

ou de sel physiologiquement admissible d'un tel disaccharide.

**4.** Utilisation conforme à l'une des revendications précédentes, pour laquelle le glycosaminoglycanne est :

a) une héparine,
b) ou un chondroïtine-sulfate A, un chondroïtine-sulfate C, un chondroïtine-sulfate D, un chondroïtine-sulfate E, un acide hyaluronique, un kératane-sulfate ou un héparane-sulfate.

**5.** Utilisation conforme à l'une des revendications précédentes, pour laquelle on utilise un sel de sodium d'héparine ou d'héparine-sulfate.

**6.** Utilisation conforme à l'une des revendications précédentes, pour laquelle l'ADNase I :

a) présente la séquence d'acides aminés donnée en tant que Séquence N° 2, ou est une ADNase I dont la séquence d'acides aminés est identique, pour plus de 65 %, à la séquence d'acides aminés donnée en tant que Séquence N° 2 et qui conserve son activité d'hydrolyse ;
b) est une ADNase I de type humain ;
c) et/ou est une ADNase I recombinante.

**7.** Utilisation conforme à l'une des revendications précédentes, pour laquelle le glycosaminoglycanne ou le sel de glycosaminoglycanne :

a) présente une masse molaire moyenne de 12 à 18 kDa ;
b) possède une activité anti-coagulante ;
c) et/ou n'a pas subi de fragmentation et/ou de dépolymérisation.

**8.** Utilisation conforme à l'une des revendications précédentes, pour laquelle le rapport de la quantité de glycosaminoglycanne ou de sel de glycosaminoglycanne à la quantité d'ADNAse I administrée vaut de 5000 unités pour 1 unité à 1 unité pour 50 unités.

**9.** Utilisation conforme à la revendication 8, pour laquelle ledit rapport vaut de 25 pour 1 à 1 pour 25.

**10.** Utilisation conforme à l'une des revendications précédentes, pour laquelle

a) le glycosaminoglycanne ou le sel de glycosaminoglycanne et l'ADNAse I sont administrés en même temps ;
b) ou le glycosaminoglycanne ou le sel de glycosaminoglycanne est administré avant ou après l'ADNAse I ;
c) et/ou le glycosaminoglycanne ou le sel de glycosaminoglycanne et l'ADNAse I sont administrés en tant que médicaments distincts.

**11.** Utilisation conforme à l'une des revendications précédentes, pour laquelle le sujet présente un volume expiratoire forcé $VEF_1$ qui est inférieur à 75 % de la valeur attendue pour un sujet équivalent, mais indemne du trouble.

**12.** Utilisation conforme à l'une des revendications précédentes, pour laquelle le glycosaminoglycanne ou le sel de glycosaminoglycanne et/ou l'ADNAse I sont administrés par inhalation, par voie intranasale et/ou par instillation.

**13.** Composition pharmaceutique comprenant :

- un glycosaminoglycanne, ou un sel physiologiquement admissible de glycosaminoglycanne, qui présente une masse molaire moyenne de 8 à 40 kDa,
- une ADNase I,
- et un excipient, véhicule ou diluant pharmacologiquement admissible.

**14.** Composition pharmaceutique conforme à la revendication 13, qui se présente sous forme de poudre sèche.

**15.** Produits comprenant un glycosaminoglycanne, ou un sel physiologiquement admissible d'un glycosaminoglycanne, lequel glycosaminoglycanne ou son sel présente une masse molaire moyenne de 8 à 40 kDa, et une ADNase I, pour utilisation dans le traitement d'un trouble **caractérisé par** la présence d'ADN extracellulaire endogène chez le sujet à traiter, étant entendu que le glycosaminoglycanne ou son sel renforce l'activité de l'ADNase I, que ces produits sont destinés à être administrés simultanément, séparément ou séquentiellement, et que le trouble est

a) une maladie respiratoire **caractérisée par** la présence d'ADN extracellulaire endogène dans le poumon,
b) choisi parmi une mucoviscidose, une limitation chronique du débit respiratoire et une pneumonie,
c) un lupus érythémateux aigu disséminé,
d) ou un trouble **caractérisé par** la présence d'ADN extracellulaire endogène au niveau d'un site d'inflammation.

**16.** Produits pour utilisation, conformes à la revendication 15, dans lesquels :

A) le glycosaminoglycanne ou le sel physiologiquement admissible de glycosaminoglycanne comporte des motifs répétés de disaccharide de formule générale (1) :

-[A-B]- (1)

dans laquelle

- chaque symbole A représente un reste, identique ou différent, de formule (i) ou (ii) :

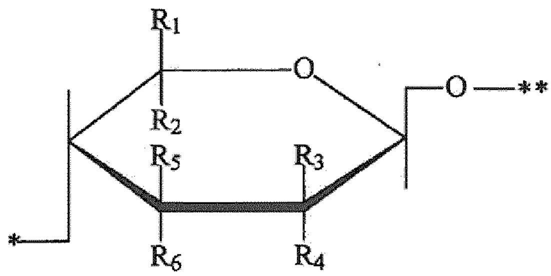

(i)

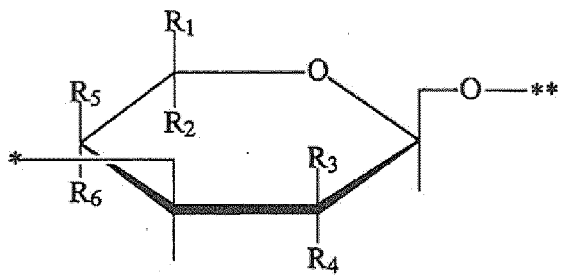

(ii)

dans lesquelles

- l'un des symboles $R_1$ et $R_2$ représente un atome d'hydrogène, et l'autre, un groupe de formule $-CO_2H$, $-SO_3H$, ou $-CH_2OR$ où R représente un atome d'hydrogène ou un groupe de formule $-SO_3H$,
- l'un des symboles $R_3$ et $R_4$ représente un atome d'hydrogène, et l'autre, un groupe de formule -OR où R représente un atome d'hydrogène ou un groupe de formule $-SO_3H$,
- l'un des symboles $R_5$ et $R_6$ représente un atome d'hydrogène, et l'autre, un groupe de formule -OH,

- l'astérisque simple * indique une liaison directe vers un atome d'hydrogène ou un reste B adjacent,
- et l'astérisque double ** indique une liaison directe vers un reste B adjacent,
- et chaque symbole B représente un reste, identique ou différent, de formule (iii) ou (iv) :

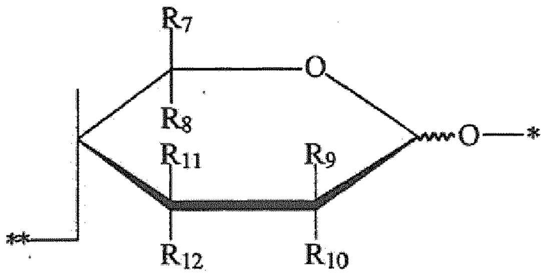

(iii)

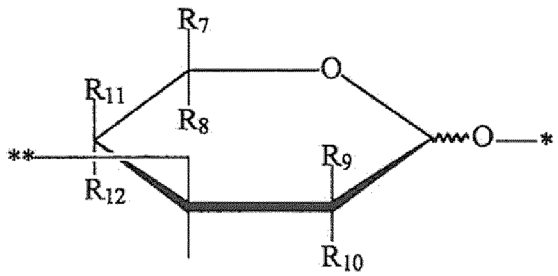

(iv)

dans lesquelles

- l'un des symboles $R_7$ et $R_8$ représente un atome d'hydrogène, et l'autre, un groupe de formule $-CH_2OH$ ou $-CH_2OSO_3H$,
- l'un des symboles $R_9$ et $R_{10}$ représente un atome d'hydrogène, et l'autre, un groupe de formule -NHAc, $-NH_2$ ou $-NHSO_3H$,
- l'un des symboles $R_{11}$ et $R_{12}$ représente un atome d'hydrogène, et l'autre, un groupe de formule -OH ou $-OSO_3H$,
- la ligne brisée ∿ indique une liaison dans l'une ou l'autre orientation stéréochimique,
- l'astérisque simple * indique une liaison directe vers un atome d'hydrogène ou un reste A adjacent,
- et l'astérisque double ** indique une liaison directe vers un reste A adjacent ;

ou de sel physiologiquement admissible d'un tel disaccharide ;

B) ou le glycosaminoglycanne ou le sel de glycosaminoglycanne est une héparine ;
C) ou le glycosaminoglycanne ou le sel de glycosaminoglycanne est un chondroïtine-sulfate A, un chondroïtine-sulfate C, un chondroïtine-sulfate D, un chondroïtine-sulfate E, un acide hyaluronique, un kératane-sulfate ou un héparane-sulfate ;
D) ou c'est un sel de sodium d'héparine ou d'héparine-sulfate qui est utilisé ;
E) ou le glycosaminoglycanne ou le sel de glycosaminoglycanne présente une masse molaire moyenne de 12 à 18 kDa ;
F) ou le glycosaminoglycanne ou le sel de glycosaminoglycanne possède une activité anti-coagulante ;
G) ou le glycosaminoglycanne ou le sel de glycosaminoglycanne n'a pas subi de fragmentation et/ou de dépolymérisation ;
H) ou l'ADNase I présente la séquence d'acides aminés donnée en tant que Séquence N° 2, ou est une ADNase I dont la séquence d'acides aminés est identique, pour plus de 65 %, à la séquence d'acides aminés donnée en tant que Séquence N° 2 ;
I) ou l'ADNase I est une ADNase I de type humain ;
J) ou l'ADNase I est une ADNase I recombinante ;
K) ou le rapport de la quantité de glycosaminoglycanne ou de sel de glycosaminoglycanne à la quantité d'ADNAse I administrée vaut de 5000 unités pour 1 unité à 1 unité pour 50 unités ;
L) ou le rapport de la quantité de glycosaminoglycanne ou de sel de glycosaminoglycanne à la quantité d'ADNAse

I administrée vaut de 25 unités pour 1 unité à 1 unité pour 25 unités.

**17.** Agent pour utilisation dans le traitement d'un sujet atteint d'un trouble **caractérisé par** la présence d'ADN extracellulaire endogène, lequel agent comprend un glycosaminoglycanne, ou un sel physiologiquement admissible d'un glycosaminoglycanne, étant entendu que le glycosaminoglycanne ou son sel présente une masse molaire moyenne de 8 à 40 kDa, que le sujet est également traité avec une ADNase I, que le glycosaminoglycanne ou son sel renforce l'activité de l'ADNase I, et que le trouble est

a) une maladie respiratoire **caractérisée par** la présence d'ADN extracellulaire endogène dans le poumon,
b) choisi parmi une mucoviscidose, une limitation chronique du débit respiratoire et une pneumonie,
c) un lupus érythémateux aigu disséminé,
d) ou un trouble **caractérisé par** la présence d'ADN extracellulaire endogène au niveau d'un site d'inflammation.

**18.** Agent pour utilisation dans le traitement d'un sujet atteint d'un trouble **caractérisé par** la présence d'ADN extracellulaire endogène, lequel agent est une ADNase I, étant entendu que le sujet est également traité avec un glycosaminoglycanne ou un sel physiologiquement admissible d'un glycosaminoglycanne, que le glycosaminoglycanne ou son sel présente une masse molaire moyenne de 8 à 40 kDa, que le glycosaminoglycanne ou son sel renforce l'activité de l'ADNase I, et que le trouble est :

a) une maladie respiratoire **caractérisée par** la présence d'ADN extracellulaire endogène dans le poumon,
b) choisi parmi une mucoviscidose, une limitation chronique du débit respiratoire et une pneumonie,
c) un lupus érythémateux aigu disséminé,
d) ou un trouble **caractérisé par** la présence d'ADN extracellulaire endogène au niveau d'un site d'inflammation.

**19.** Composition pharmaceutique conforme à la revendication 13 ou 14, ou agent pour utilisation, conforme à la revendication 17 ou 18, dans laquelle ou lequel :

A) le glycosaminoglycanne ou le sel physiologiquement admissible de glycosaminoglycanne comporte des motifs répétés de disaccharide de formule générale (1) :

-[A-B]- (1)

dans laquelle

- chaque symbole A représente un reste, identique ou différent, de formule (i) ou (ii) :

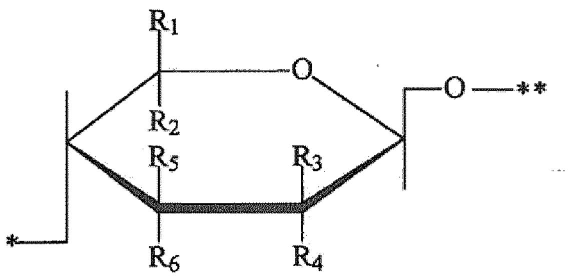

(i)

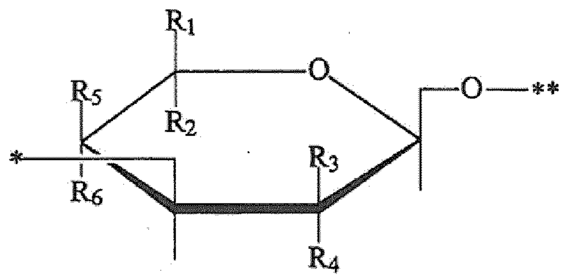

(ii)

dans lesquelles

- l'un des symboles $R_1$ et $R_2$ représente un atome d'hydrogène, et l'autre, un groupe de formule $-CO_2H$, $-SO_3H$, ou $-CH_2OR$ où R représente un atome d'hydrogène ou un groupe de formule $-SO_3H$,
- l'un des symboles $R_3$ et $R_4$ représente un atome d'hydrogène, et l'autre, un groupe de formule -OR où R représente un atome d'hydrogène ou un groupe de formule $-SO_3H$,
- l'un des symboles $R_5$ et $R_6$ représente un atome d'hydrogène, et l'autre, un groupe de formule -OH,
- l'astérisque simple * indique une liaison directe vers un atome d'hydrogène ou un reste B adjacent,
- et l'astérisque double ** indique une liaison directe vers un reste B adjacent,
- et chaque symbole B représente un reste, identique ou différent, de formule (iii) ou (iv) :

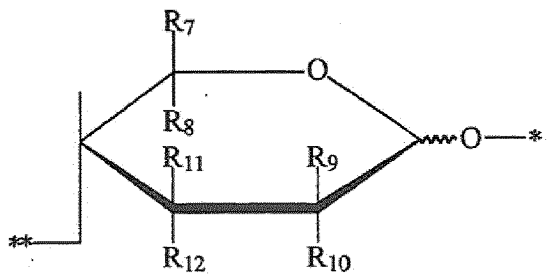

(iii)

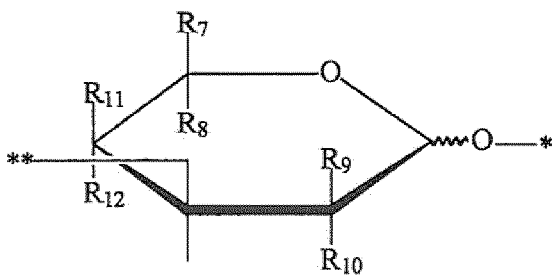

(iv)

dans lesquelles

- l'un des symboles $R_7$ et $R_8$ représente un atome d'hydrogène, et l'autre, un groupe de formule $-CH_2OH$ ou $-CH_2OSO_3H$,
- l'un des symboles $R_9$ et $R_{10}$ représente un atome d'hydrogène, et l'autre, un groupe de formule -NHAc, $-NH_2$ ou $-NHSO_3H$,
- l'un des symboles $R_{11}$ et $R_{12}$ représente un atome d'hydrogène, et l'autre, un groupe de formule -OH ou $-OSO_3H$,
- la ligne brisée ∿∿ indique une liaison dans l'une ou l'autre orientation stéréochimique,
- l'astérisque simple * indique une liaison directe vers un atome d'hydrogène ou un reste A adjacent,
- et l'astérisque double ** indique une liaison directe vers un reste A adjacent ;

ou de sel physiologiquement admissible d'un tel disaccharide ;

B) ou le glycosaminoglycanne ou le sel de glycosaminoglycanne est une héparine ;
C) ou le glycosaminoglycanne ou le sel de glycosaminoglycanne est un chondroïtine-sulfate A, un chondroïtine-sulfate C, un chondroïtine-sulfate D, un chondroïtine-sulfate E, un acide hyaluronique, un kératane-sulfate ou un héparane-sulfate ;
D) ou c'est un sel de sodium d'héparine ou d'héparine-sulfate qui est utilisé ;
E) ou le glycosaminoglycanne ou le sel de glycosaminoglycanne présente une masse molaire moyenne de 12 à 18 kDa ;
F) ou le glycosaminoglycanne ou le sel de glycosaminoglycanne possède une activité anti-coagulante ;
G) ou le glycosaminoglycanne ou le sel de glycosaminoglycanne n'a pas subi de fragmentation et/ou de dépolymérisation ;
H) ou l'ADNase I présente la séquence d'acides aminés donnée en tant que Séquence N° 2, ou est une ADNase I dont la séquence d'acides aminés est identique, pour plus de 65 %, à la séquence d'acides aminés donnée en tant que Séquence N° 2 ;
I) ou l'ADNase I est une ADNase I de type humain ;
J) ou l'ADNase I est une ADNase I recombinante ;
K) ou le rapport de la quantité de glycosaminoglycanne ou de sel de glycosaminoglycanne à la quantité d'ADNAse I administrée vaut de 5000 unités pour 1 unité à 1 unité pour 50 unités ;
L) ou le rapport de la quantité de glycosaminoglycanne ou de sel de glycosaminoglycanne à la quantité d'ADNAse I administrée vaut de 25 unités pour 1 unité à 1 unité pour 25 unités.

**20.** Nébuliseur ou autre dispositif à aérosol liquide, inhalateur à poudre sèche ou inhalateur doseur, contenant une composition comprenant :

- un glycosaminoglycanne, ou un sel physiologiquement admissible de glycosaminoglycanne, qui présente une masse molaire moyenne de 8 à 40 kDa,
- une ADNase I,
- et un excipient, véhicule ou diluant pharmacologiquement admissible.

**21.** Utilisation d'un glycosaminoglycanne, ou d'un sel physiologiquement admissible de glycosaminoglycanne, lequel glycosaminoglycanne ou son sel présente une masse molaire moyenne de 8 à 40 kDa, pour renforcer l'activité d'une ADNase I *in vitro.*

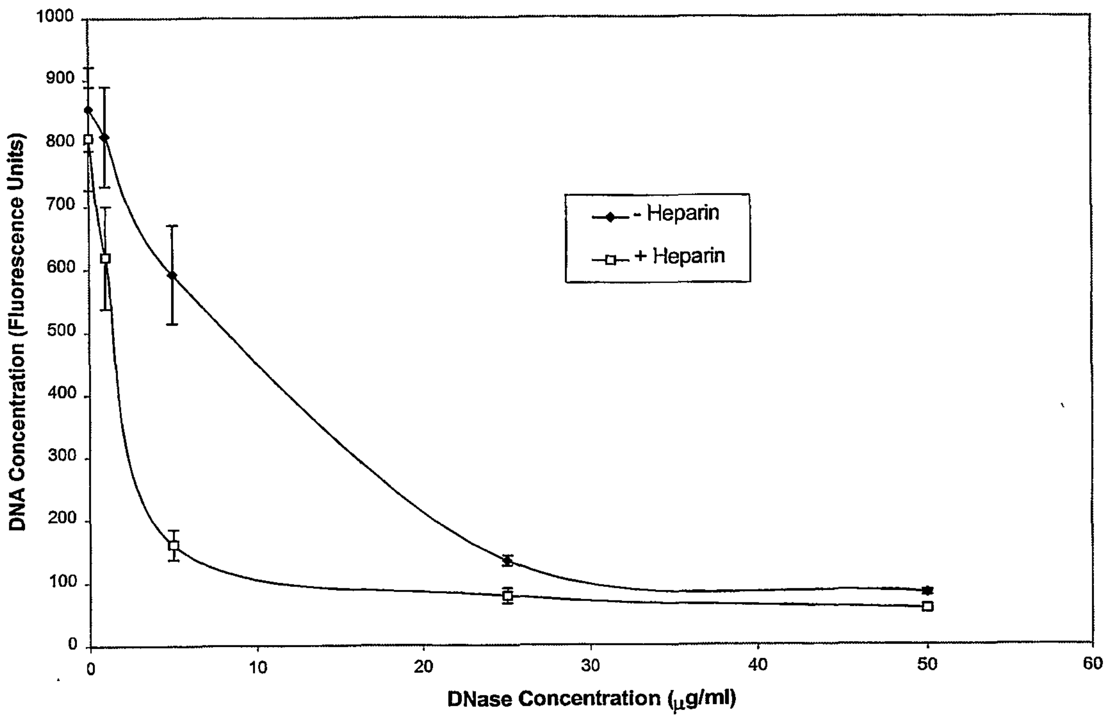

1000
900
800
700
600
500
400
300
200
100
0
0
10
20
30
40
50
60
DNA Concentration (Fluorescence Units)
DNase Concentration (μg/ml)
- Heparin
+ Heparin


**Figure 1**

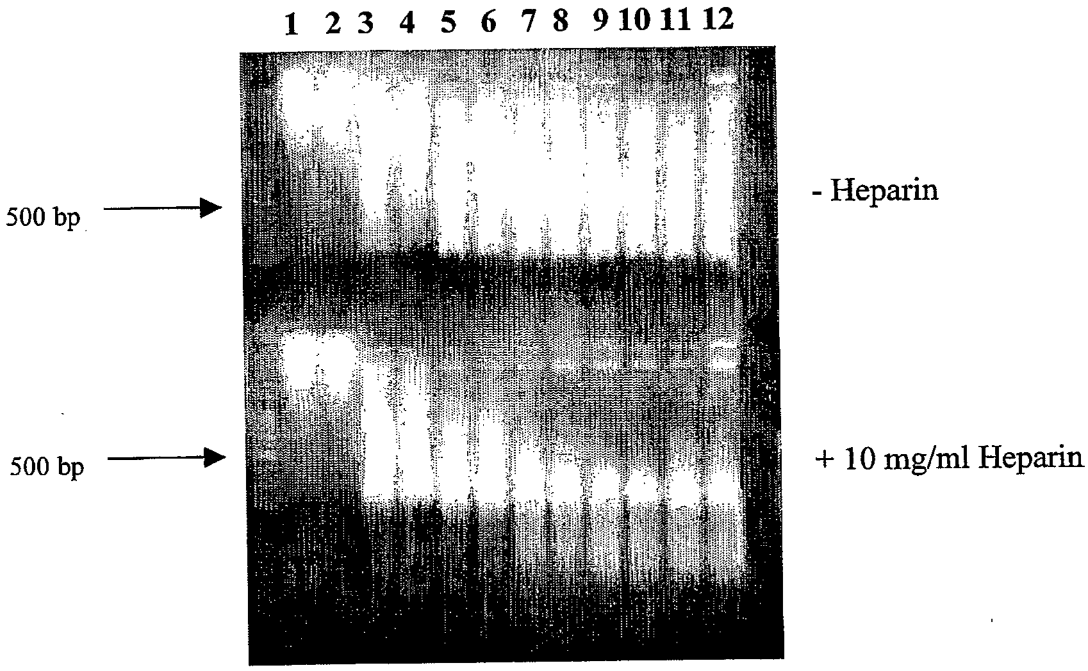


**Figure 2**

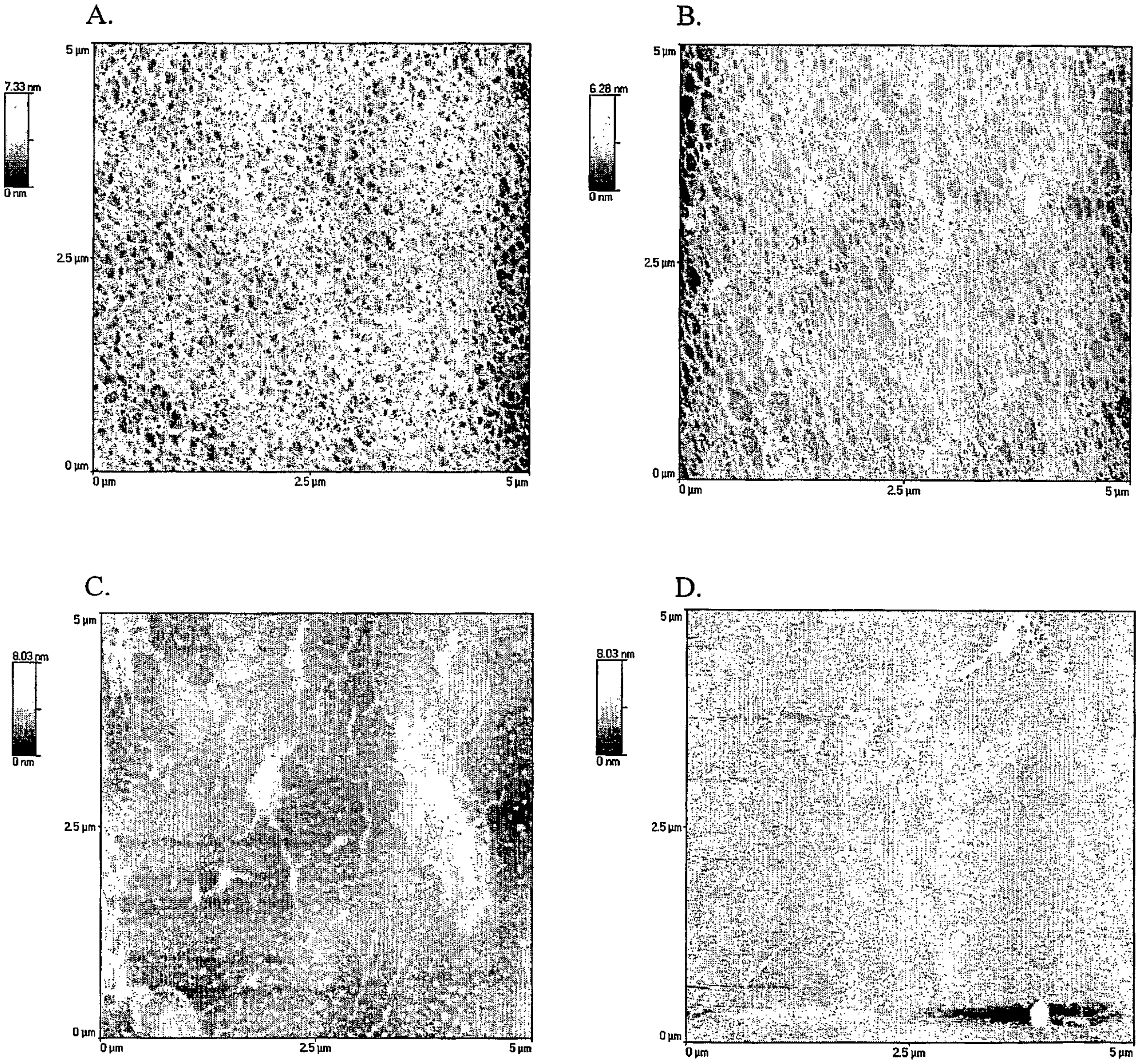
A.
7.33 nm
0 nm
5 µm
2.5 µm
0 µm
0 µm
2.5 µm
5 µm
B.
6.28 nm
0 nm
5 µm
2.5 µm
0 µm
0 µm
2.5 µm
5 µm
C.
8.03 nm
0 nm
5 µm
2.5 µm
0 µm
0 µm
2.5 µm
5 µm
D.
8.03 nm
0 nm
5 µm
2.5 µm
0 µm
0 µm
2.5 µm
5 µm

**Figure 3**

A.

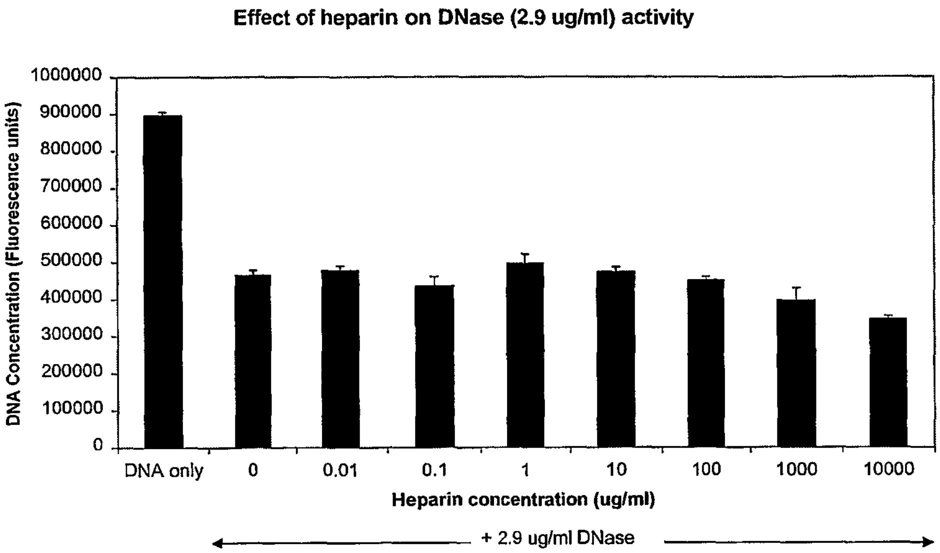
Effect of heparin on DNase (2.9 ug/ml) activity
DNA Concentration (Fluorescence units)
1000000
900000
800000
700000
600000
500000
400000
300000
200000
100000
0
DNA only
0
0.01
0.1
1
10
100
1000
10000
Heparin concentration (ug/ml)
+ 2.9 ug/ml DNase

B.

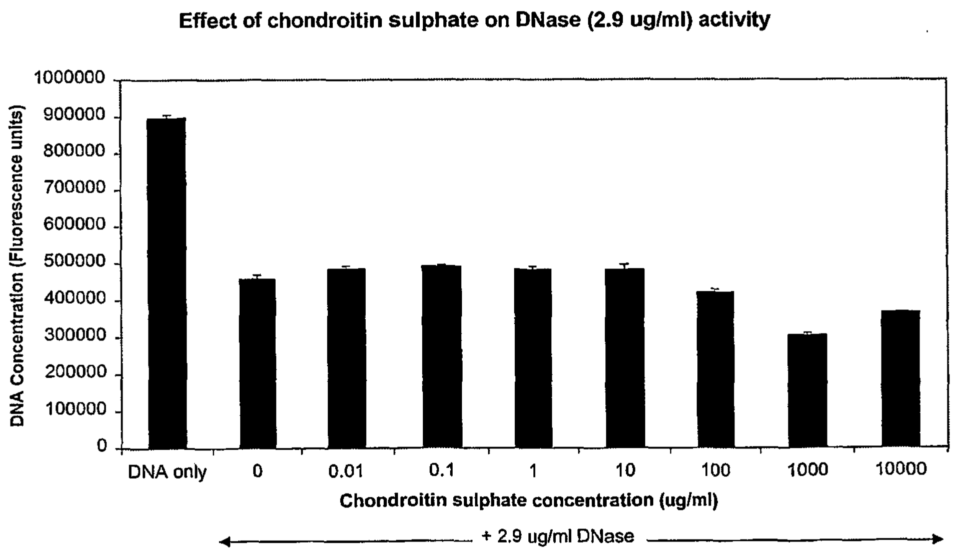
Effect of chondroitin sulphate on DNase (2.9 ug/ml) activity
DNA Concentration (Fluorescence units)
1000000
900000
800000
700000
600000
500000
400000
300000
200000
100000
0
DNA only
0
0.01
0.1
1
10
100
1000
10000
Chondroitin sulphate concentration (ug/ml)
+ 2.9 ug/ml DNase

# Figure 4

C.

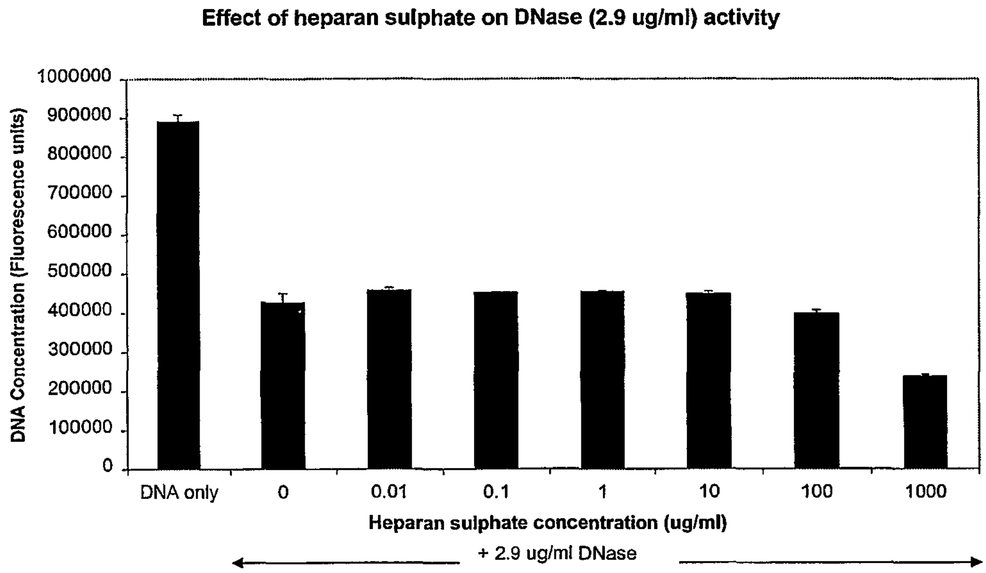


D.

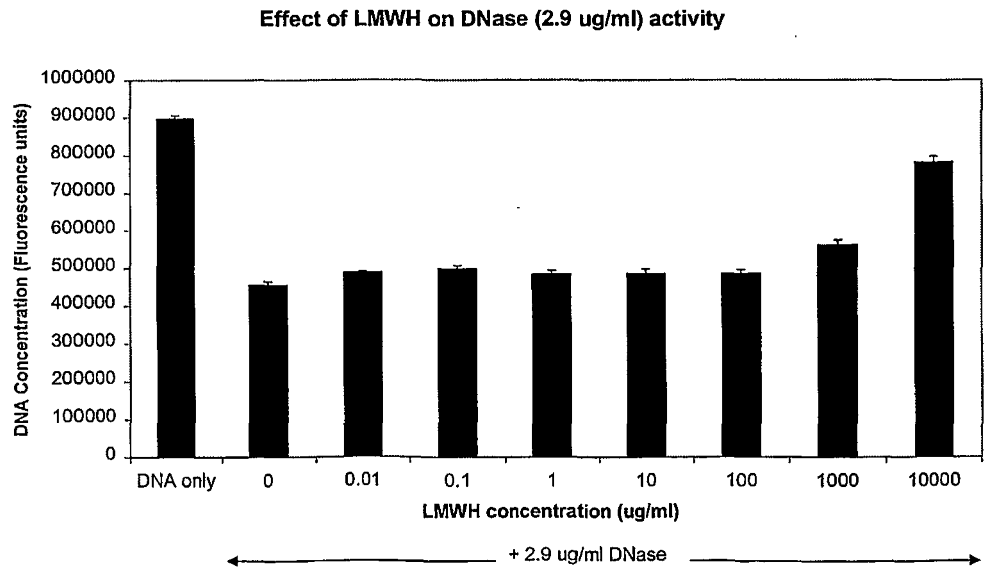


**Figure 4 (continued)**

E.

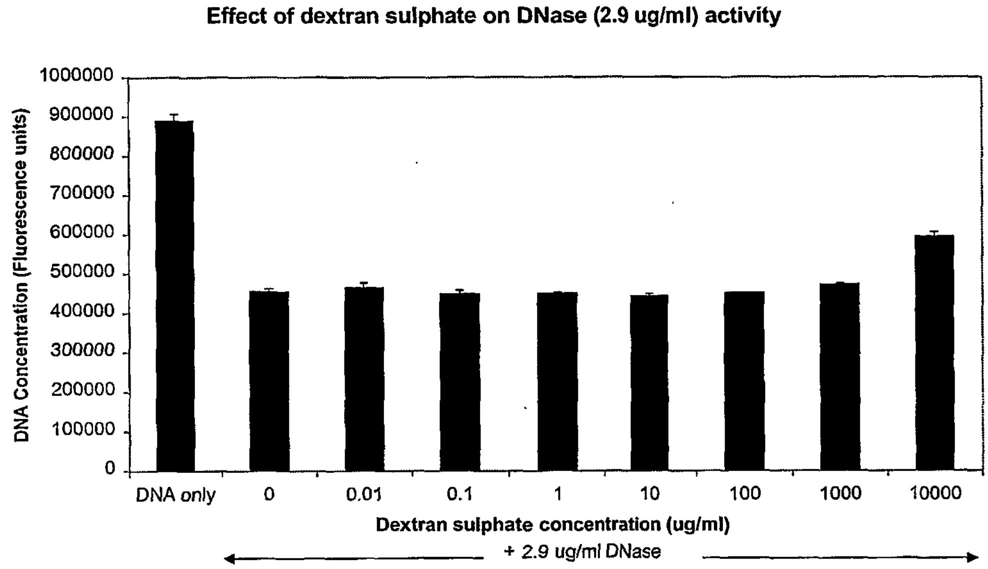


**Figure 4 (continued)**

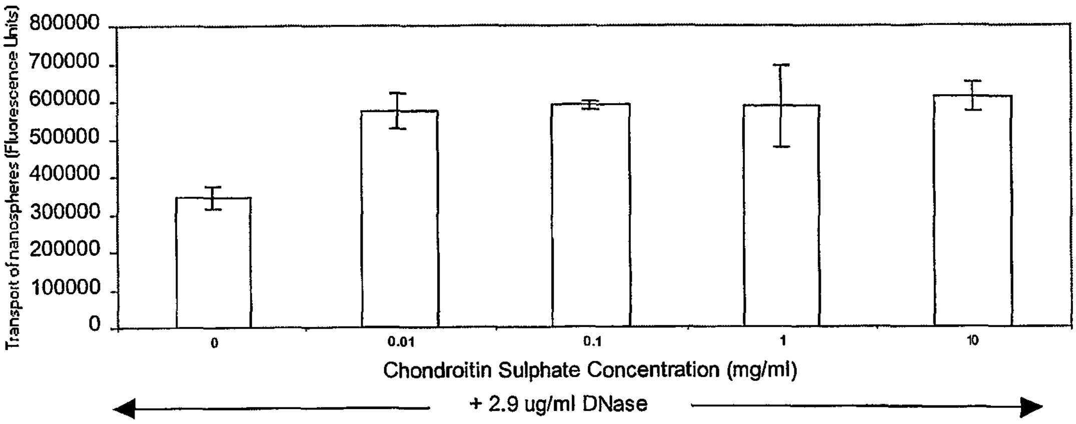
Transport of nanospheres (Fluorescence Units)
800000
700000
600000
500000
400000
300000
200000
100000
0
0
0.01
0.1
1
10
Chondroitin Sulphate Concentration (mg/ml)
+ 2.9 ug/ml DNase

Figure 5

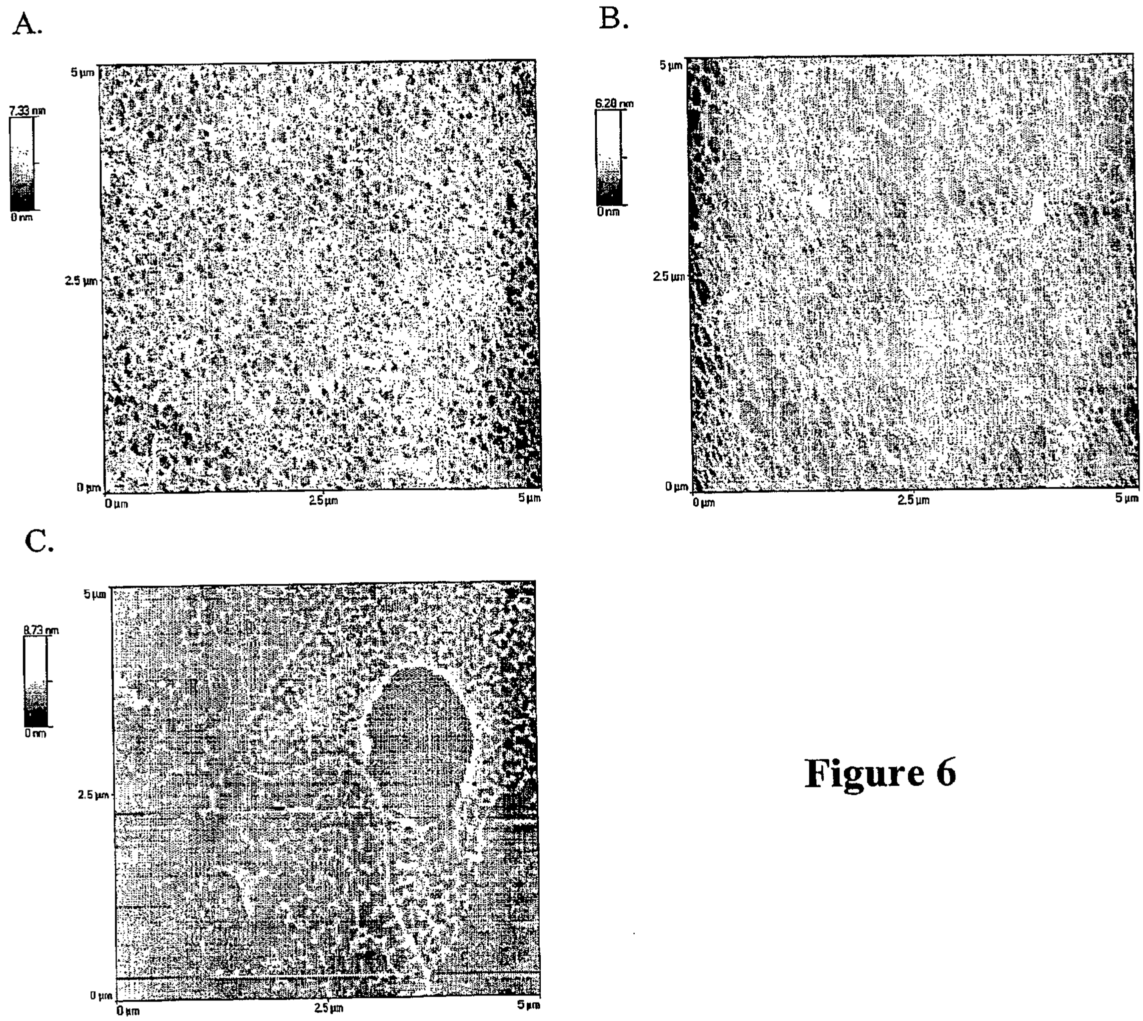

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9410567 A
- US 6348343 B


### Non-patent literature cited in the description


- **LABARCE ; PAIDEN.** *Anal. Biochem.,* 1980, vol. 102, 344-352
- **KUNITZ.** *J. Gen. Physiol.,* 1950, vol. 33, 349-362
- **KUNITZ.** *J. Gen. Physiol.,* 1950, vol. 33, 363-377
- **KUMICK.** *Arch. Biochem.,* 1950, vol. 29, 41-53
- **SINICROPI et al.** *Anal. Biochem.,* 1994, vol. 222, 351-358
- **JANMEY.** *J. Biochem. Biophys. Methods,* 1991, vol. 22, 41-53
- **DEVEREUX et al.** *Nucleic Acids Research,* 1984, vol. 12, 387-395
- **ALTSCHUL S. F.** *J Mol Evol,* 1993, vol. 36, 290-300
- **ALTSCHUL, S, F et al.** *J Mol Biol,* 1990, vol. 215, 403-10
- **HENIKOFF ; HENIKOFF.** *Proc. Natl. Acad. Sci. USA,* 1992, vol. 89, 10915-10919
- **KARLIN ; ALTSCHUL.** *Proc. Natl. Acad. Sci. USA,* 1993, vol. 90, 5873-5787
- **MANNHERZ et al.** *Eur. J. Biochem.,* 1980, vol. 104, 367-379
- *Thorax,* 1997, vol. 52 (5), 1-28
- *European Resp. Journal,* 1993, vol. 6 (16
- Coates, Lung Function,: Assessment and Applications In Medicine. Blackwell, 1969
- Remington's Pharmaceutical Sciences. Mack Publishing Company
- **LABARCA ; PAIDER.** *Anal. Biochem.,* 1980, vol. 102, 344-352